(19) Europäisches Patentamt
European Patent Office
Office européen des brevets

(11) Veröffentlichungsnummer : **0 044 573**
**B1**

(12) **EUROPÄISCHE PATENTSCHRIFT**

(45) Veröffentlichungstag der Patentschrift : **29.02.84**

(51) Int. Cl.³ : **C 07 D455/02**, A 61 K 31/435// C07D211/76

(21) Anmeldenummer : **81106675.2**

(22) Anmeldetag : **11.10.79**

(60) Veröffentlichungsnummer der früheren Anmeldung nach Art. 76 EPÜ : **0012170**

(54) **7-Phenylchinolizine.**

(30) Priorität : **13.10.78 CH 10654/78**
**03.08.79 CH 7156/79**

(43) Veröffentlichungstag der Anmeldung : **27.01.82 Patentblatt 82/04**

(45) Bekanntmachung des Hinweises auf die Patenterteilung : **29.02.84 Patentblatt 84/09**

(84) Benannte Vertragsstaaten : **BE CH DE FR GB IT LU NL SE**

(56) Entgegenhaltungen :
**JOURNAL OF MEDICINAL CHEMISTRY, Band 18, Nr. 11, veröffentlicht in 1975, Seiten 1126-1130 Washington, U.S.A. M.E. ROGERS et al.: "3-Arylquinolizidines, Potential Antidepressant Agents"**
**JOURNAL OF MEDICINAL CHEMISTRY, Band 12, Nr. 4, veröffentlicht in Juli 1969, Seiten 563-560 Washington, U.S.A. A.H. BECKETT et al.: "Bridgehead nitrogen compounds as potential analgetics"**

(73) Patentinhaber : **F. HOFFMANN-LA ROCHE & CO. Aktiengesellschaft**
**CH-4002 Basel (CH)**

(72) Erfinder : **Imhof, René, Dr.**
**Bleumatthöhe 3**
**CH-5264 Gipf-Oberfrick (CH)**
Erfinder : **Kyburz, Emilio, Dr.**
**Unterer Rebbergweg 127**
**CH-4153 Reinach (CH)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

EP 0 044 573 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

7-Phenylchinolizine

Die vorliegende Erfindung betrifft neue Phenyl-chinolizine der allgemeinen Formel

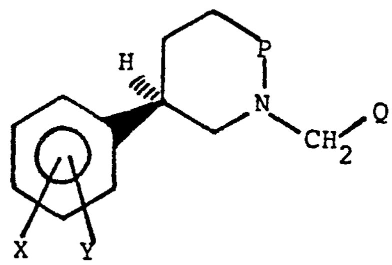

in der X Wasserstoff, Fluor, Chlor, $C_{1-6}$-Alkoxy, $C_{1-6}$-Alkyl oder Trifluormethyl und Y Wasserstoff, Fluor, Chlor, $C_{1-6}$-Alkoxy oder $C_{1-6}$-Alkyl bedeuten und P und Q zusammen eine der Gruppen

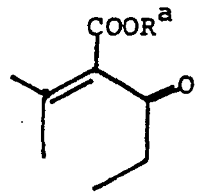
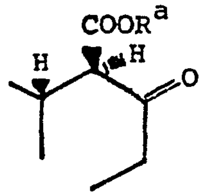
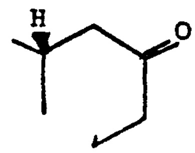
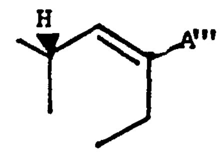
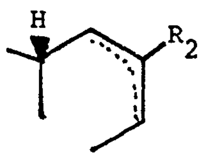
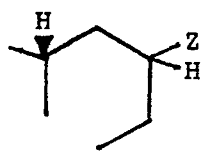
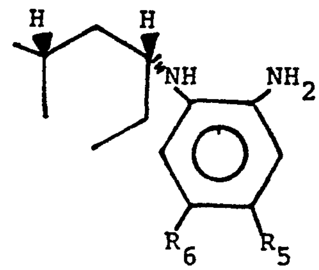

darstellt, worin $R^a$ nieder-Alkyl, $R^2$ $C_{1-6}$-Alkyl, gegebenenfalls durch Halogen, $C_{1-6}$-Alkoxy oder Trifluormethyl substituiertes Phenyl, $R^5$ und $R^6$ unabhängig voneinander Wasserstoff, Methyl, Fluor, Chlor, Methoxy, Trifluormethyl oder zusammen $—(CH_2)_4—$, Z eine abspaltbare Gruppe und A''' eine Gruppe mit der Bedeutung

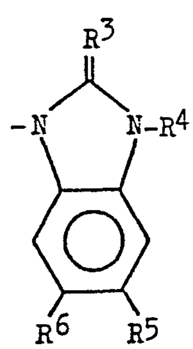

oder

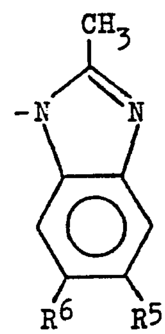

mit folgender Bedeutung für $R^3$-$R^6$:

$R^3$ : Sauerstoff oder Schwefel,

$R^4$ : Wasserstoff oder $C_{1-6}$-Alkyl,

$R^5$ und $R^6$ unabhängig voneinander : Wasserstoff, Methyl, Fluor, Chlor, Methoxy, Trifluormethyl oder zusammen $—(CH_2)_4—$, wobei falls $R^4$ nieder-Alkyl bedeutet, $R^3$ Sauerstoff darstellt, bedeuten, in Form des Racemats oder der Enantiomeren. Diese Phenylchinolizine sind verwendbar als Zwischenprodukte bei der Herstellung von Phenyl-chinolizidinen der allgemeinen Formel

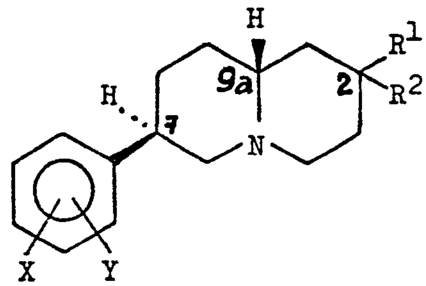

(I)

worin X, Y, $R^1$ und $R^2$ folgendes bedeuten :

X : Wasserstoff, Fluor, Chlor, nieder-Alkoxy, nieder-Alkyl oder Trifluormethyl,

Y : Wasserstoff, Fluor, Chlor, nieder-Alkoxy oder nieder-Alkyl, vorzugsweise Wasserstoff,

$R^1$ : Hydroxy, nieder-Alkoxy, Acyloxy oder, falls $R^2$ die nachstehend definierte Gruppe A darstellt, Wasserstoff,

$R^2$ : nieder-Alkyl, gegebenenfalls durch Halogen, nieder-Alkoxy oder Trifluormethyl substituiertes Phenyl, oder (falls $R^1$ = Wasserstoff) eine Gruppe A mit der Bedeutung

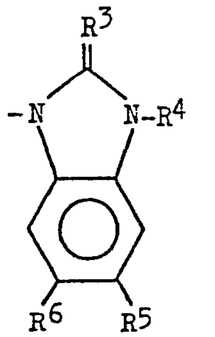

oder

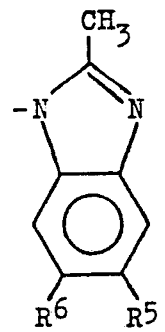

(A)

mit folgender Bedeutung für $R^3$-$R^6$ :

$R^3$ : Sauerstoff oder Schwefel,

$R^4$ : Wasserstoff oder nieder-Alkyl,

$R^5$ und $R^6$ unabhängig voneinander : Wasserstoff, Methyl, Fluor, Chlor, Methoxy, Trifluormethyl oder zusammen $-(CH_2)_4-$, in Form des Racemats oder der Enantiomeren, sowie in Form der freien Basen wie von Säureadditionssalzen.

Wie in der Formel I und in verschiedenen der nachfolgenden Formeln angegeben, befinden sich die Wasserstoffatome in den Stellungen 7 und 9a transständig zueinander, wobei in den Formeln das 9a-Wasserstoffatom willkürlich in β-Stellung und das 7-Wasserstoffatom dementsprechend in α-Stellung angegeben ist. Die Chinolizidine der Formel I wie auch die entsprechenden Zwischenprodukte und Ausgangsstoffe sind jedoch nicht auf diese absolute Konfiguration beschränkt, sondern umfassen auch die entsprechenden enantiomeren Formen, also Verbindungen mit Wasserstoff in 9aα und 7β, sowie die Racemate dieser beiden enantiomeren Formen. Dasselbe gilt mit Bezug auf die Konfiguration in 2-Stellung des Chinolizidins. Sofern, wie beispielsweise in der nachfolgenden Formel III die 2- und 9a-Wasserstoffatome in β-Stellung und das 7-Wasserstoffatom in α-Stellung angegeben ist, so soll damit lediglich die relative Konfiguration an diesen 3 Asymmetriezentren festgelegt werden (9aH zu 7H : trans ; 9aH zu 2H : cis), jedoch keine Beschränkung auf das eine Enantiomere ausgesprochen sein.

Unter nieder-Alkyl- bzw. nieder-Alkoxygruppen sind im vorliegenden Rahmen solche Gruppen zu verstehen, die 1-6, insbesondere 1-4 Atome enthalten, wobei die Gruppen mit 3 und mehr C-Atomen geradkettig oder verzweigt sein können. Die als möglicher $R^1$-Substituent genannte Acyloxy-Gruppe kann sich von organischen, gesättigten, gegebenenfalls substituierten Carbonsäuren, insbesondere niedern, gegebenenfalls halogensubstituierten Alkancarbonsäuren, wie der Essigsäure oder der Trifluoressigsäure, ableiten.

Als Säureadditionssalze der Phenylchinolizidine der Formel I kommen die pharmakologisch verträglichen Salze mit den üblicherweise zur Salzbildung verwendeten organischen und anorganischen Säuren in Betracht. Beispiele solcher Säuren sind : Mineralsäuren, wie Schwefelsäure, Salpetersäure, Phosphorsäure, Halogenwasserstoffsäuren (z. B. Salzsäure, Bromwasserstoffsäure) ; organische Säuren, wie Weinsäure, Citronensäure, aliphatische oder aromatische Sulfonsäuren, Maleinsäure, Mandelsäure etc. Die Salzbildung kann auf an sich bekannte Art erfolgen.

Die Formel I umfasst folgende Untergruppen :

a) die Alkyl- resp. Aryl-carbinole der Formel

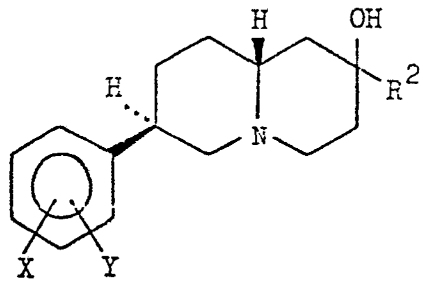

(II)

bevorzugt diejenigen der Formel

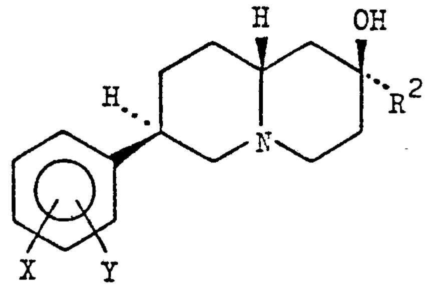

(II-1)

wobei X, Y und $R^2$ dasselbe wie oben bedeuten, in racemischer Form und in Form der Enantiomeren, sowie gegebenenfalls in Form von Säureadditionssalzen,

b) die Ester und Aether der sub a) genannten Carbinole, d. h. die Verbindungen der Formel

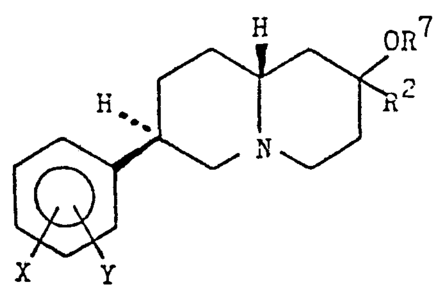

(IV)

worin X, Y und $R^2$ dasselbe wie oben bedeuten und $R^7$ nieder-Alkyl oder Acyl darstellt, in racemischer Form und in Form der Enantiomeren, sowie gegebenenfalls in Form von Säureadditionssalzen,

c) die Benzimidazolinon (und -thion)-Derivate der Formel

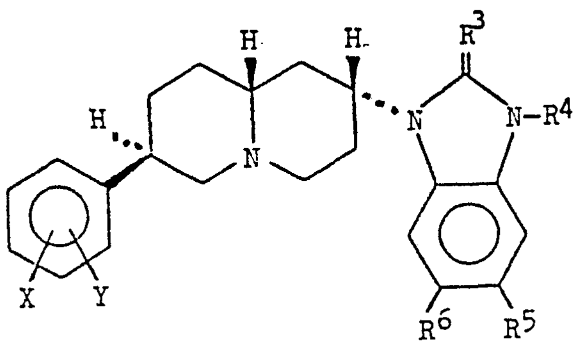

(III-1)

worin X, Y sowie $R^3$-$R^6$ dasselbe wie oben bedeuten, in racemischer Form und in Form der Enantiomeren, sowie gegebenenfalls in Form von Säureadditionssalzen.

d) die Methylbenzimidazol-Derivate der Formel

(Siehe Figur (III-2), Seite 5 f.)

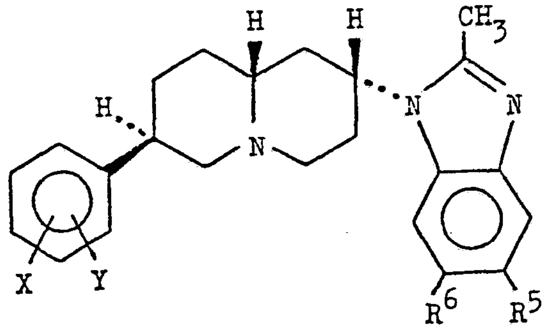

(III-2)

mit obiger Bedeutung für X, Y, $R^5$ und $R^6$ in racemischer Form und in Form der Enantiomeren, sowie gegebenenfalls in Form von Säureadditionssalzen.

Bevorzugte Verbindungen der Formel II-1 sind diejenigen, in denen $R^2$ verzweigtes nieder-Alkyl, X Fluor oder Chlor und Y Wasserstoff, Fluor oder Chlor, wobei X und Y in ortho- und/oder para-Stellung stehen, dies vorwiegend wegen ihrer neuroleptischen Wirkung. Besonders bevorzugte Verbindungen der Formel II-1 sind diejenigen, in denen $R^2$ tert. Butyl, X o-Chlor und Y p-Chlor oder $R^2$ tert. Butyl, X p-Trifluormethyl und Y Wasserstoff bedeuten.

Die Verbindungen der Formel IV sind vorwiegend wegen ihrer analgetischen Wirkung bevorzugt. Darunter besonders bevorzugt sind diejenigen, in denen

a) X und Y Wasserstoff, $OR^7$ gleich wie der 9a-Wasserstoff orientiertes Acetoxy und $R^2$ n-Butyl oder

b) X und Y Wasserstoff, $OR^7$ gleich wie der 9a-Wasserstoff orientiertes Trifluoracetoxy und $R^2$ tert. Butyl oder

c) X o-Chlor, $R^2$ wie der 9a-Wasserstoff orientiertes Phenyl und $OR^7$ Acetoxy bedeuten.

Die Verbindungen der Formeln III-1 und III-2 sind vorwiegend neuroleptisch wirksam. Bevorzugt sind die Benzimidozolinon (bzw. -thion)-Derivate der Formel III-1, in denen X Wasserstoff, o-Fluor oder o-Chlor, Y Wasserstoff, $R^3$ Sauerstoff oder Schwefel, $R^4$ Wasserstoff, $R^5$ Fluor, Chlor oder Trifluormethyl und $R^6$ Wasserstoff, Chlor oder Fluor darstellen. Ferner sind die folgenden Verbindungen der Formel III-1 besonders bevorzugt:

a) X = o-Cl ; Y, $R^4$, $R^5$, $R^6$ = H ; $R^3$ = O
b) X = o-Cl ; Y, $R^4$ = H ; $R^3$ = O ; $R^5$, $R^6$ = $CH_3$
c) X = o-Cl ; Y, $R^4$ = H ; $R^3$ = O ; $R^5$, $R^6$ = Cl
d) X = o-Cl ; Y, $R^5$, $R^6$ = H ; $R^3$ = O ; $R^4$ = $CH_3$
e) X = o-Cl ; Y, $R^4$, $R^6$ = H ; $R^5$ = $CF_3$ ; $R^3$ = O
f) X = o-Cl ; Y, $R^4$, $R^6$ = H ; $R^5$ = Cl ; $R^3$ = O
g) X = o-Cl ; Y, $R^4$, $R^6$ = H ; $R^5$ = Cl ; $R^3$ = S
h) X = o-Cl ; Y, $R^4$ = H ; $R^3$ = S ; $R^5$, $R^6$ = Cl
i) X = o-Cl ; Y, $R^4$ = H ; $R^3$ = O ; $R^5$ und $R^6$ zusammen $-(CH_2)_4-$
j) X = o-F ; Y, $R^4$ = H ; $R^3$ = O ; $R^5$, $R^6$ = $CH_3$
k) X = o-F ; Y, $R^4$ = H ; $R^3$ = O ; $R^5$, $R^6$ = Cl
l) X = o-F ; Y, $R^4$ = H ; $R^3$ = S ; $R^5$, $R^6$ = Cl
m) X = o-F ; Y, $R^4$ = H ; $R^3$ = O ; $R^5$ und $R^6$ zusammen $-(CH_2)_4-$
n) X = o-F ; Y, $R^4$, $R^6$ = H ; $R^5$ = $CF_3$ ; $R^3$ = O
o) X = o-F ; Y, $R^4$, $R^6$ = H ; $R^5$ = $CF_3$ ; $R^3$ = S
p) X = o-F ; Y, $R^4$, $R^6$ = H ; $R^5$ = Cl ; $R^3$ = O
q) X = o-F ; Y, $R^4$, $R^6$ = H ; $R^5$ = Cl ; $R^3$ = S.

Die Phenyl-chinolizidine der Formel I mit den oben angegebenen Bedeutungen für X, Y, $R^1$ und $R^2$ können dadurch hergestellt werden, dass man

1. zur Herstellung von Verbindungen der allgemeinen Formel

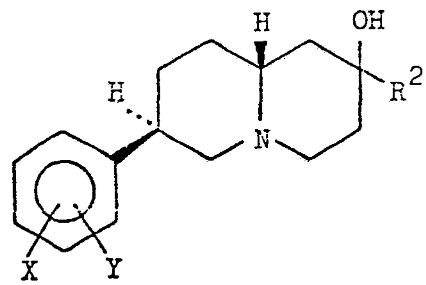

(II)

worin X, Y und $R^2$ dasselbe wie oben bedeuten, entweder a) ein Keton der allgemeinen Formel V

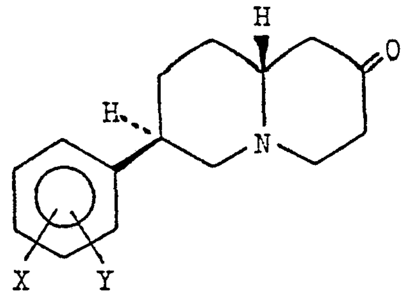

(V)

worin X und Y dasselbe wie oben bedeuten, mit einer metallorganischen, den Rest $R^2$ liefernden Verbindung umsetzt, und gegebenenfalls das erhaltene Gemisch der Diastereomeren auftrennt, oder b) an die $\Delta^1$- bzw. $\Delta^2$-Doppelbindung einer Verbindung der allgemeinen Formel

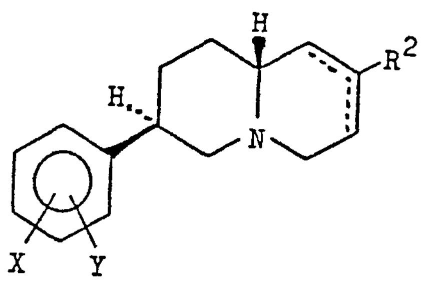

(VI)

worin X, Y und $R^2$ dasselbe wie oben bedeuten und die gestrichelte Bindung eine Doppelbindung in 1,2- oder 2,3-Stellung darstellt, durch Umsetzung mit einem Hydratisierungsmittel Wasser anlagert, oder dass man

2. zur Herstellung von Verbindungen der allgemeinen Formel

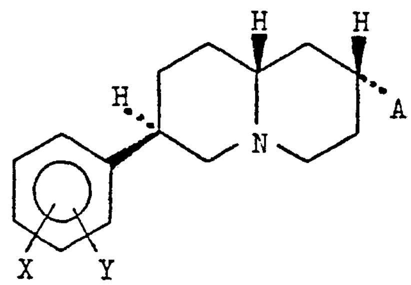

(III)

worin X, Y und A dasselbe wie oben bedeuten, eine Verbindung der allgemeinen Formel

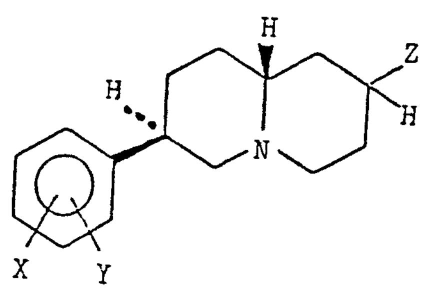

(VII)

worin X und Y dasselbe wie oben bedeuten und Z eine abspaltbare Gruppe darstellt, mit einer Verbindung der allgemeinen Formel

H—A (VIII)

worin A dasselbe wie oben bedeutet, umsetzt, oder dass man

3. zur Herstellung einer Verbindung der allgemeinen Formel

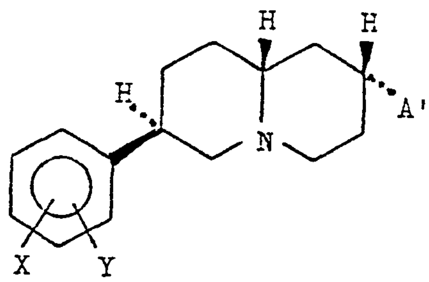

(III-3)

worin X und Y dasselbe wie oben bedeuten und A' wie A ist, wobei jedoch $R^3$ Sauerstoff darstellt, eine Verbindung der allgemeinen Formel

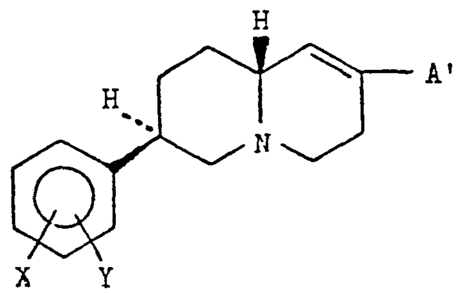

(IX)

worin X, Y und A' dasselbe wie oben bedeuten, katalytisch hydriert, oder dass man

4. zur Herstellung einer Verbindung der allgemeinen Formel

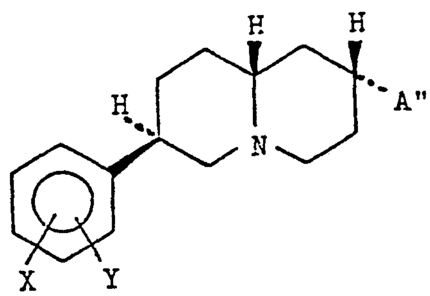

(III-4)

worin X und Y dasselbe wie oben bedeuten und A" wie A ist, wobei jedoch $R^3$ Sauerstoff und $R^4$ Wasserstoff darstellt, ein Phenylen-diamin der allgemeinen Formel

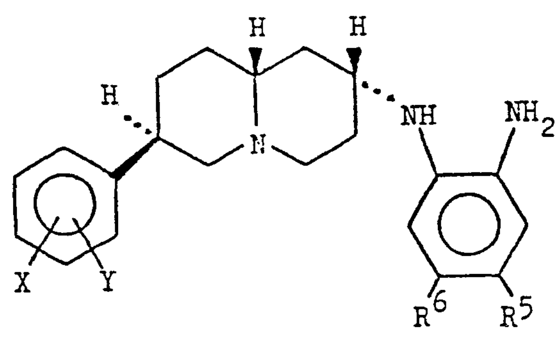

(X)

worin X, Y, $R^5$ und $R^6$ dasselbe wie oben bedeuten, mit einem Cyclisierungsmittel der allgemeinen Formel

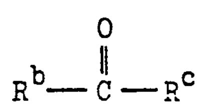

(XI)

worin $R^b$ und $R^c$ Halogen, Amino oder 1-Imidazolyl bedeuten, oder mit Essigsäure oder einem Orthoessigsäureester kondensiert, oder dass man

5. zur Herstellung von Verbindungen der allgemeinen Formel

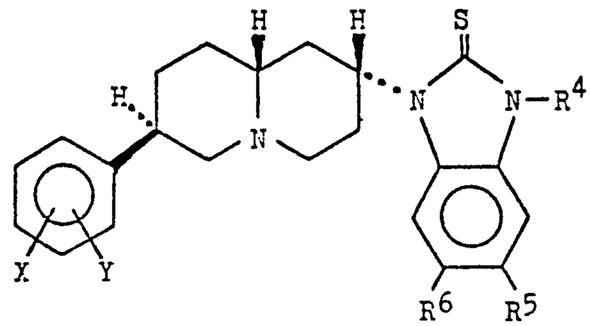

(III-5)

worin X, Y sowie $R^4$-$R^6$ dasselbe wie oben bedeuten, entweder a) eine entsprechende Oxo-verbindung der allgemeinen Formel

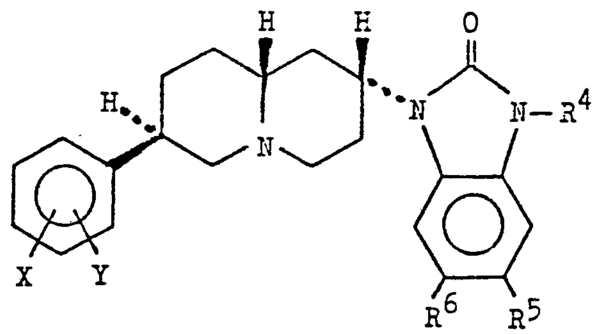

(III-6)

worin X, Y sowie $R^4$-$R^6$ dasselbe wie oben bedeuten, mit $P_2S_5$ umsetzt, oder b) ein Phenylen-diamin der obigen allgemeinen Formel X mit Thiophosgen, Thiocarbonyldimidazol oder $CS_2$ umsetzt und anschliessend gegebenenfalls N-alkyliert, oder dass man

6. zur Herstellung von Verbindungen der allgemeinen Formel

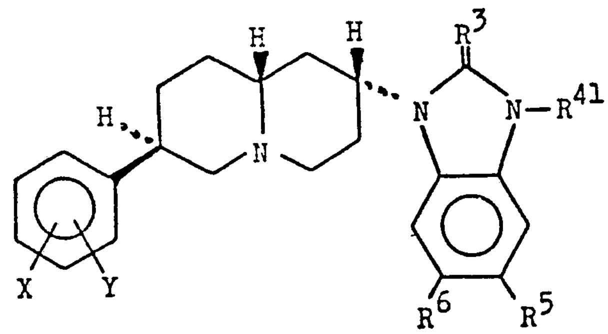

(III-7)

worin X, Y, $R^3$, $R^5$ und $R^6$ dasselbe wie oben bedeuten und $R^{41}$ nieder-Alkyl darstellt, eine Verbindung der allgemeinen Formel

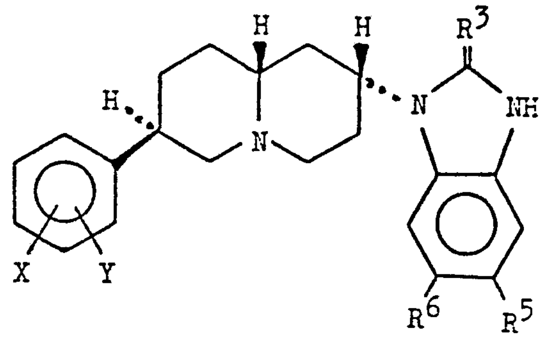

(III-8)

worin X, Y, $R^3$, $R^5$ und $R^6$ dasselbe wie oben bedeuten, N-alkyliert, oder dass man

7. zur Herstellung von Aethern und Estern der allgemeinen Formel

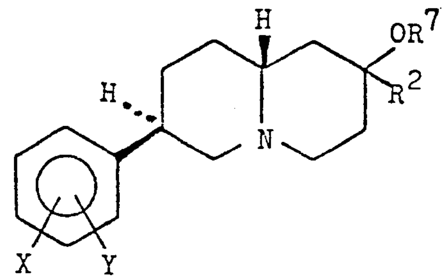

(IV)

worin X, Y und $R^2$ dasselbe wie oben bedeuten und $R^7$ nieder-Alkyl oder Acyl darstellt, einen Alkohol der obigen allgemeinen Formel II mit einem den Rest $R^7$ abgebenden Verätherungs- bzw. Veresterungsmittel umsetzt und dass man erhaltene Racemate gegebenenfalls spaltet und erhaltene Basen gegebenenfalls in Säureadditionssalze überführt.

Sämtliche der in den vorstehenden Verfahrensvarianten 1-7 genannten Reaktionen können nach an sich bekanten Methoden durchgeführt werden.

Zur Ueberführung eines Ketons V in ein tertiäres Carbinol II gemäss der Verfahrensvariante 1a) können die üblichen, den Rest $R^2$ liefernden metallorganischen Verbindungen, wie die entsprechenden Grignard-verbindungen $R^2$Mg-Halogen, $R^2$-Lithium-verbindungen oder $R^2$-Lithium-Kupfer-verbindungen verwendet werden.

Mit der Carbinolbildung aus dem Keton V tritt ein drittes Asymmetriezentrum (in 2-Stellung) auf. Der neu eintretende Substituent $R^2$ kann dabei mit Bezug auf das 9a-Wasserstoffatom die cis- und/oder trans-Position einnehmen. So werden z. B. bei der im nachfolgenden Beispiel 3 beschriebenen Umsetzung eines racemischen Ketons V zwei diastereomere Produkte II (je als Racemat) erhalten, während bei der im nachstehenden Beispiel 1 beschriebenen Umsetzung eines racemischen Ketons V von den beiden theoretisch möglichen diastereomeren Racematen II praktisch nur das eine Racemat und zwar dasjenige mit $R^2$ in trans-Position zum 9a-Wasserstoff, wie dies durch die Formel II-1 ausgedrückt wird, erhalten wird.

Erhaltene Diastereomeren-Gemische wie auch erhaltene Racemate lassen sich nach an sich bekannten Methoden auftrennen bzw. spalten.

Zur Anlagerung von Wasser an die $\Delta^1$- bzw. $\Delta^2$-Doppelbindung von Verbindungen der Formel VI gemäss der Verfahrensvariante 1b) kann als Hydratisierungsmittel beispielsweise wässerige Schwefelsäure benützt werden.

Zur Herstellung der Benzimidazol-Derivate III gemäss der Verfahrensvariante 2 kann man eine Verbindung VII, die als abspaltbare Gruppe Z z. B. Cl, Br, Mesyloxy oder Tosyloxy enthält, mit einer Verbindung VIII, vorzugsweise in Form eines Alkalimetallsalzes, umsetzen.

Zur Herstellung der Benzimidazolderivate III-3 nach der Verfahrensvariante 3 kann man die $\Delta^1$-Doppelbindung einer Verbindung IX katalytisch hydrieren, wobei als Katalysatoren übliche Hydrierungskatalysatoren, wie $PtO_2$ oder Pd/C (z. B. 10 %ig) verwendet werden können.

Zur Herstellung der Benzimidazolderivate III-4 nach der Verfahrensvariante 4 kann man ein Phenylendiamin X mit einem Cyclisierungsmittel XI, wie z. B. Phosgen, Carbonyldiimidazol oder Harnstoff, kondensieren, wobei ein Benzimidazolinon-Derivat des Typus III-1 entsteht, oder auch mit Essigsäure oder einem Orthoessigsäureester, z. B. dem Trimethyl- oder Triäthylester, wobei ein Methylbenzimidazol Derivat des Typus III-2 erhalten wird.

Zur Herstellung der Thione III-3 kann man entweder gemäss Variante 5a nach an sich bekannten Methoden den Sauerstoff des Imidazolrings einer Oxo-verbindung III-6 mit $P_2S_5$ gegen Schwefel austauschen, oder gemäss Variante 5b auf an sich bekannte Art ein Phenylendiamin X mit Thiophosgen, Thiocarbonyldiimidazol oder $CS_2$ umsetzen. Die anschliessende, fakultative N-Alkylierung an $R^4$ erfolgt wie nachstehend für die Verfahrensvariante 6 angegeben.

Die Verfahrensvarianten 4 und 5b eignen sich besonders gut zur Herstellung von Verbindungen III-4 bzw. III-5, worin $R^5$ von $R^6$ verschieden ist.

Zur Herstellung von im Imidazolring N-alkylierten Verbindungen III-7 nach der Verfahrensvariante 6 kann man ein übliches N-Alkylierungsmittel, wie ein nieder-Alkylhalogenid, z. B. Methyljodid, verwenden.

Zur Herstellung der Aether und Ester IV nach der Verfahrensvariante 7 kann man auf an sich bekannte Art ein Carbinol II, z. B. durch Umsetzung mit einem Säureanhydrid $(R^7CO)_2O$ in Gegenwart von Pyridin oder mit einem Isopropenylester $CH_2 = C(CH_3)O—CO—R^7$ in Gegenwart von p-Toluolsulfonsäure, verestern bzw. durch Umsetzung mit einem nieder-Alkylhalogenid (basische Verätherung) oder einem $R^7OH$/Schwefelsäure-Gemisch (saure Verätherung) veräthern.

Die im folgenden als Zwischenprodukte bzw. Ausgangsstoffe genannten Verbindungen können, soweit sie nicht bekannt sind, nach an sich bekannten Methoden hergestellt werden. So können z. B. die in der Verfahrensvariante 1a) als Ausgangsmaterial verwendeten Ketone V auf an sich bekannte Art durch Decarboxylierung von β-Keto-estern der allgemeinen Formel XIV mit der in dieser Formel angegebenen relativen Konfiguration bezüglich der Positionen 1, 7 und 9a

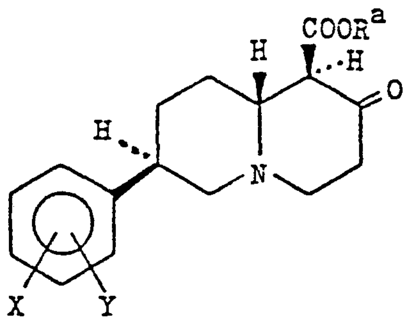

(XIV)

worin X und Y dasselbe wie oben bedeuten und $R^a$ nieder-Alkyl darstellt, erhalten werden (vgl. z. B. Beispiel 1b).

Die β-Keto-ester XIV können ihrerseits aus den entsprechenden ungesättigten β-Keto-estern der allgemeinen Formel XIII

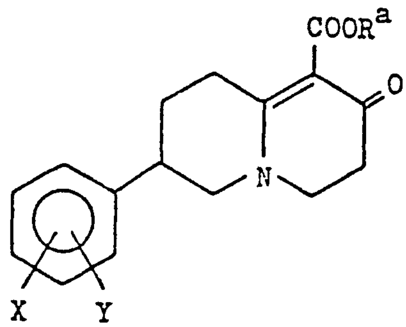

(XIII)

worin X, Y und $R^a$ dasselbe wie oben bedeuten, auf an sich bekannte Art durch Hydrierung erhalten werden (vgl. z. B. das Beispiel 1c).

Die Ester XIII können aus entsprechenden im Phenyl gegebenenfalls substituierten 5-Phenyl-2-piperidonen nach an sich bekannten Methoden (vgl. z. B. das Beispiel 1d) erhalten werden.

Die in der Verfahrensvariante 6 als Ausgangsmaterial genannten Benzimidazoline III-8 können aus den entsprechenden ungesättigten Verbindungen der allgemeinen Formel XV (mit der in dieser Formel angegebenen relativen Konfiguration in den Stellungen 7 und 9a)

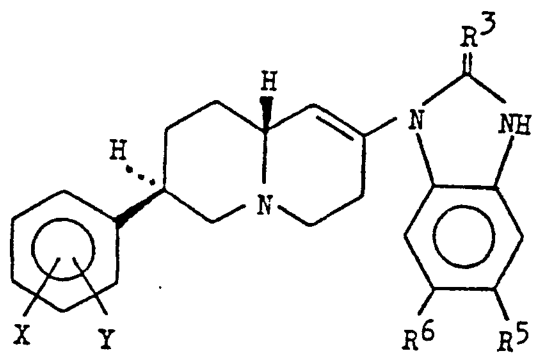

(XV)

worin X, Y, $R^3$, $R^5$ und $R^6$ dasselbe wie oben bedeuten, durch Hydrierung erhalten werden.

Durch N-Alkylierung von XV wie in Verfahrensvariante 6 erhält man N-alkylierte Ausgangsverbindungen IX.

Die Verbindungen XV können ihrerseits aus den bereits genannten β-Keto-estern XIV nach dem in Beispiel 7b beschriebenen Verfahren bzw. in Analogie dazu erhalten werden.

Die Verbindungen VI und VII können nach dem in den Beispielen 13 und 14 beschriebenen Verfahren bzw. in Analogie dazu erhalten werden.

Die neuen Zwischenprodukte bzw. Ausgangsstoffe der Formeln V, VI, VII, IX, X, XIII, XIV und XV sind Gegenstand der vorliegenden Erfindung.

Die Phenyl-chinolizidine der Formel I zeichnen sich durch wertvolle pharmakologische Eigenschaften aus. Sie sind neuroleptisch, zentraldämpfend, tranquillisierend, antiemetisch und/oder analgetisch wirksam und können demgemäss als Antipsychotica bzw. Antiemetica bzw. Analgetica bei der Bekämpfung von Psychosen und Neurosen bzw. von Uebelkeit bzw. von Schmerz Verwendung finden.

Die neuroleptischen Eigenschaften der Verbindungen wurden zahlenmässig mit den nachstehend beschriebenen Tests ermittelt :

« Pole Climbing » Test (Ratte)

Zur Versuchsdurchführung wurde pro Dosis eine Gruppe von 10 Ratten eingesetzt, die daraufhin trainiert worden waren, durch Hinaufspringen an eine vertikale isolierte Stange im Versuchskäfig, dem unmittelbar nach einem akustischen Signal (konditionierter Reiz), via Bodengitter ausgelösten Elektroschock (unkonditionierter Reiz) zu entkommen. Die Hemmung der konditionierten Reaktion bei 50 % der Tiere während einer Beobachtungszeit von 6 Stunden wird durch den Parameter ED 50 BCR, die Hemmung der unkonditionierten Reaktion durch den Parameter ED 10 BUR bestimmt.

Spiroperidol-binding Test (in vitro)

In den in vitro Versuchen wurde Kalbsstriatum als Rezeptor verwendet (Lit. : Creese et al., Life Sci. *17,* 993 (1975) und analog der von Fields et al., Brain Research *136,* 578 (1977) beschriebenen Methoden mit [$^3$H] Spiroperidol inkubiert. Mit einem Computerprogramm wurde aus 4 verschiedenen Konzentrationen in dreifacher Ausführung die IC 50 bestimmt, welche die Konzentration der Testsubstanz angibt, bei welcher eine 50 %ige Ablösung der spezifischen Bindung von [$^3$H] Spiroperidol am Rezeptor eintritt.

In der nachstehenden Tabelle A sind einige Versuchsresultate zusammengestellt.

Tabelle A

| Verbindung | Pole-Climbing BCR $ED_{50}$ i. p. (mg/kg) | Pole-Climbing BUR $ED_{10}$ i. p. (mg/kg) | Spiroperidol-Binding $IC_{50}$ (nanomolar) |
|---|---|---|---|
| rac-(9aβH)-2α-tert.-Butyl-7β-(2,4-dichlorphenyl)octahydro-2H-chinolizin-2-ol · HCl | 6,4 | 5,4 | 79 |
| (−)-(2S,7R,9aR)-2-tert.-Butyl-7-(2,4-dichlorphenyl) octahydro-2H-chinolizin-2-ol · HCl | 4,7 | 3,8 | 41 |
| rac-1-[(9aβH)-7β-(o-Chlorphenyl)-octahydro-2H-chinolizin-2α-yl]-5,6-dimethyl-2-benzimidazolinon · HCl | 1,75 | 4,55 | 5 |
| rac-1-[(9aβH)-7β-(o-Chlorphenyl)-octahydro-2H-chinolizin-2α-yl]-5-trifluormethyl-2-benzimidazolinon · HCl | 0,21 | 0,41 | 10 |
| rac-1-[(9aβH)-7β-(o-Fluorphenyl)-octahydro-2H-chinolizin-2α-yl]-5-chlor-2-benzimidazolinon · HCl | 0,064 | 0,175 | 3,1 |

Die analgetischen Eigenschaften der Verbindungen wurden zahlenmässig mit den nachstehenden beschriebenen Tests ermittelt :

Writhing Test (Maus)

Zur Durchführung der Versuche wurden 8 männliche Mäuse (20-22 g) pro Dosis eingesetzt. 60 Minuten nach erfolgter oraler Verabreichung der Testsubstanz wurde den Tieren 10 ml/kg der Testlösung intraperitoneal injiziert. Anschliessend an eine Latenzzeit von 5 Minuten wurde die Zahl der Tiere registriert, bei denen während 5 Minuten nicht mehr als 1 charakteristisches Writhing-Symptom (konvulsive Streckbewegung des Körpers) auftrat. Die ED 50 gibt diejenige Dosis an, bei welcher 50 % der Tiere nicht mehr als 1 Writhing zeigen.

Hot-plate Test (Maus)

8 Mäuse (20-22 g) wurden pro Dosis eingesetzt. Die Tiere wurden 60 Minuten nach oraler Gabe der Testsubstanz auf eine erwärmte Metallplatte von 60 ± 0,5 °C gesetzt. Unbehandelte Mäuse begannen innerhalb von max. 10 Sekunden ihre Pfoten zu lecken. Die ED 50 ist als jene Dosis definiert, bei der 50 % der Tiere mit dem Pfotenlecken erst nach einer Latenzzeit von mehr als 10 Sekunden beginnen.

In der nachstehenden Tabelle B sind einige Versuchsresultate zusammengestellt :

(Siehe Tabelle B, Seite 12 f.)

Tabelle B

| Verbindung | Writhing 60′ $ED_{50}$ p. o. mg/kg | Hot-plate 60′ $ED_{50}$ p. o. mg/kg |
|---|---|---|
| (+)-2-Butyloctahydro-7-phenyl-2H-chinolizin-2-yl-acetat · HCl | 0,19 | 2,6 |
| rac-(9aβH)-2α-n-Butyl-7β-phenyl-octahydro-2H-chinolizin-2-yl-acetat · HCl | 0,75 | 25 |
| rac-(9aβH)-2α-tert.-Butyl-7β-phenyl-octahydro-2H-chinolizin-2-yl-trifluoracetat · HCl | 8,7 | 86 |
| rac-(9aβH)-2α-Phenyl-7β-(m-methoxyphenyl)-octahydro-2H-chinolizin-2-yl-propionat · HCl | 2,8 | 12 |
| rac-(9aβH)-2β-Phenyl-7β-(o-chlorphenyl)-octahydro-2H-chinolizin-2-yl-acetat · HCl | 116 | 284 |

Die Verbindungen der Formel I in Form des Racemats oder der Enantiomeren, sowie in Form der freien Basen wie von Säureadditionssalzen können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, lokal oder perkutan, z. B. in Form von Salben, Crèmes, Gelées, Lösungen, oder parenteral, z. B. in Form von Injektionslösungen, erfolgen.

Zur Herstellung von Tabletten, Lacktabletten, Dragées und Hartgelatinekapseln können die erfindungsgemässen Verbindungen mit pharmazeutisch inerten, anorganischen oder organischen Excipientien verabreicht werden. Als solche Excipientien kann man z. B. für Tabletten, Dragées und Hartgelatinekapseln Lactose, Maisstärke oder Derivate davon, Talk, Stearinsäure oder deren Salze etc. verwenden. Für Weichgelatinekapseln eignen sich als Excipientien z. B. vegetabile Oele, Wachse, Fette, halbfeste und flüssige Polyole etc. ; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z. B. Wasser, Polyole, Saccharose, Invertzucker, Glukose und dergleichen. Für Injektionslösungen eignen sich als Excipientien z. B. Wasser, Alkohole, Polyole, Glyzerin, vegetabile Oele etc. Für Suppositorien, lokale oder perkutane Anwendungsformen eignen sich als Excipientien z. B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

In den genannten pharmazeutischen Präparaten kann die Dosierung etwa im Bereich von 0,5-100 mg liegen. Bei oraler Applikation kommen Wirkstoffmengen von etwa 0,05 mg/kg bis etwa 10 mg/kg pro Tag und bei parenteraler Applikation solche von etwa 0,01 mg/kg bis 1 mg/kg pro Tag in Betracht.

Beispiel 1

a) Zu einer auf – 75 °C gekühlten Lösung von tert. Butyllithium in Pentan (100 ml ; 1,2 molar) werden unter starkem Rühren 22,2 g rac-(9aβH)-7β-(o-Chlorphenyl)octahydro-2H-chinolizin-2-on, gelöst in 300 ml absolutem Tetrahydrofuran, tropfenweise unter Argon zugesetzt. Anschliessend belässt man weitere 30 Minuten bei – 75 °C und arbeitet dann wie folgt auf : Durch Zugabe von 100 ml gesättigter Ammoniumchloridlösung wird hydrolysiert und dann zwischen Essigester und Wasser verteilt. Nach nochmaliger Extraktion mit Essigester trocknet man die gesamte organische Phase über Magnesiumsulfat und dampft ein. Aus dem Rückstand kann das Endprodukt (rac-(9aβH)-2α-tert. Butyl-7β-(o-chlorphenyl)octahydro-2H-chinolizin-2-ol) aus 1 400 ml Essigester durch Einleiten von gasförmigem Chlorwasserstoff als HCl-Salz kristallisiert werden : Ausbeute 20,4 g (71 %). Smp. 276-278 °C. Als Variante zu diesem Reinigungsverfahren kann der Rückstand an Kieselgel mit Aether-Hexan = 1 : 1 chromatographiert werden.

Wenn anstelle von rac-(9aβH)-7β-(o-Chlorphenyl)octahydro-2H-chinolizin-2-on äquimolare Mengen entsprechend substituierter Ketone zur Reaktion eingesetzt werden, können folgende tert. Butylcarbinole erhalten werden :

(Siehe Tabelle 1, Seite 13 f.)

Tabelle 1

rac-(9aβH)-2α-tert. Butyl-7β-($R_a$)octahydro-2H-chinolizin-2-ol :

| | | Smp. °C |
|---|---|---|
| $R_a$ = phenyl | HCl-Salz | 230 (Zers.) |
| p-chlorphenyl | HCl-Salz | 286-288 |
| 2,4-dichlorphenyl | HCl-Salz | 270-272 |
| 2,6-dichlorphenyl | HCl-Salz | 262-263 |
| p-trifluormethylphenyl | HCl-Salz | 285-286 |

Wenn anstelle von tert. Butyllithium äquimolare Mengen analoger Alkyl- oder Aryllithium-Reagenzien mit den entsprechenden Ketonen umgesetzt werden, können folgende Carbinole erhalten werden :

Tabelle 2

rac-(9aβH)-($R_b$)-7β-($R_a$)octahydro-2H-chinolizin-2-ol :

| | | | Smp. °C |
|---|---|---|---|
| $R_b$ = 2α-sec.-Butyl | $R_a$ = 2,4-dichlorphenyl | HCl-Salz | 244-246 |
| 2α-n-Butyl | 2,4-dichlorphenyl | HCl-Salz | 227-230 |
| 2α-iso-Propyl | 2,4-dichlorphenyl | HCl-Salz | 201-205 |
| 2(α + β)-Methyl | 2,4-dichlorphenyl | HCl-Salz | 149-151 |
| 2α-n-Butyl | phenyl | Base | 100-102 |
| 2α-phenyl | o-chlorphenyl | HCl-Salz | 266-267 |
| 2β-phenyl | o-chlorphenyl | HCl-Salz | 175° amorph |
| 2α-phenyl | m-methoxyphenyl | Base | 153-155 |
| 2β-phenyl | m-methoxyphenyl | Base | 122-124 |
| 2α-phenyl | phenyl | Base | 156-158 |
| 2β-phenyl | phenyl | Base | 99-102 |
| 2α-m-Methoxyphenyl | phenyl | $^1$H-NMR | ($CDCl_3$) : 3,77(s) |
| 2β-m-Methoxyphenyl | phenyl | $^1$H-NMR | ($CDCl_3$) : 3,82(s) |
| 2α-p-chlorphenyl | phenyl | HCl-Salz | 249-250 |
| 2α-m-trifluormethylphenyl | p-chlorphenyl | Base | 119-123 |
| 2β-m-trifluormethylphenyl | p-chlorphenyl | Base | 122-124 |

b) Das als Ausgangsmaterial verwendete rac-(9aβH)-7β-(o-Chlorphenyl)octahydro-2H-chinolizin-2-on kann wie folgt erhalten werden :

76,2 g Methyl-rac-(1αH,9aβH)-7β-(o-chlorphenyl)octahydro-2-oxo-2H-chinolizin-1-carboxylat werden in 1,2 l 6N Salzsäure gelöst und 3 Stunden am Rückfluss gekocht. Die abgekühlte Reaktionslösung wird anschliessend auf Eis gegossen und mit konz. Natronlauge alkalisch gestellt. Extraktion mit 3 × 500 ml Methylenchlorid und Eindampfen der über Magnesiumsulfat getrockneten organischen Phase ergeben 68 g Rohprodukt. Aus Aether-Hexan können 50,4 g rac-(9aβH)-7β-(o-Chlorphenyl)octahydro-2H-chinolizin-2-on kristallisiert werden. Smp. 80-82 °C (81 %).

Wenn anstelle von Methyl-rac-(1αH,9aβH-7β-(o-chlorphenyl)octahydro-2-oxo-2H-chinolizin-1-carboxylat äquimolare Mengen entsprechend substituierter β-Ketoester decarboxyliert werden, erhält man folgende Produkte :

(Siehe Tabelle 3, Seite 14 f.)

Tabelle 3

rac-(9aβH)-7β-($R_a$)octahydro-2H-chinolizin-2-on :

| | | Smp. °C |
|---|---|---|
| $R_a$ = phenyl | | 71- 72 |
| p-chlorphenyl | | 80- 82 |
| p-trifluormethylphenyl | | 103-104 |
| o-methylphenyl | IR (Film) | 1 726 $cm^{-1}$ |
| m-methoxyphenyl | IR (Film) | 1 726 $cm^{-1}$ |
| 2,4-dichlorphenyl | | 116-117 |
| 2,6-dichlorphenyl | | 116-118 |
| o-fluorphenyl | | 74- 76 |

c) Das im Absatz 1b) als Ausgangsmaterial verwendete Octahydro-carboxylat kann seinerseits aus dem entsprechendem Hexahydro-carboxylat wie folgt erhalten werden :

180 g Methyl-rac-7-(o-chlorphenyl)-3,4,6,7,8,9-hexahydro-2-oxo-2H-chinolizin-1-carboxylat suspendiert man in 3,6 l Monoglym (Dimethoxyäthan), kühlt auf – 30 °C und addiert unter Rühren 1,33 l DIBAH (Diisobutylaluminiumhydrid, 20 %ige Lösung in Toluol). Anschliessend wird 1 Stunde bei – 20 bis – 30 °C gerührt und dann bei dieser Temperatur mit 1,72 l 2N Natronlauge hydrolysiert. Zur Aufarbeitung verteilt man zwischen 25 l Wasser und 20 l Chloroform ; die organische Phase wird nochmals mit 2 × 5 l Wasser gewaschen, über Magnesiumsulfat getrocknet und eingedampft : 185,2 g Rohprodukt. Aus Aether-Hexan können 109 g Produkt kristallisiert werden. Durch Chromatographie der Mutterlauge an 1 kg Kieselgel mit Essigester werden nochmals 37 g Produkt erhalten. Gesamtausbeute an Methyl-rac-(1αH, 9aβH)-7β-(o-chlorphenyl)octahydro-2-oxo-2H-chinolizin-1-carboxylat : 136 g (81 %), Smp. 122-124 °C.

Wenn anstelle von Methyl-rac-7-(o-chlorphenyl)-3,4,6,7,8,9-hexahydro-2-oxo-2H-chinolizin-1-carboxylat äquimolare Mengen entsprechend substituierter Edukte mit DIBAH reduziert werden, gelangt man zu folgenden Verbindungen :

Tabelle 4

Methyl-rac-(1αH,9aβH)-7β-($R_a$)octahydro-2-oxo-2H-chinolizin-1-carboxylat :

| | | Smp. °C |
|---|---|---|
| $R_a$ = phenyl | | 114-116 |
| p-chlorphenyl | | 102-105 |
| p-trifluormethylphenyl | | 108-111 |
| o-methylphenyl | | 94- 98 |
| m-methoxyphenyl | IR (Film) : | 1 752, 1 723, 1 660 $cm^{-1}$ |
| 2,4-dichlorphenyl | | 116-118 |
| 2,6-dichlorphenyl | | 130-131 |
| o-fluorphenyl | | 109-110 |

d) Das im Absatz 1c) als Ausgangsmaterial verwendete Hexahydro-carboxylat kann seinerseits wie folgt erhalten werden :

150 g 5-(o-Chlorphenyl)-2-piperidon werden in 2 l Methylenchlorid gelöst und zu 410 g Triäthyloxonium-tetrafluorborat (Meerweinsalz) in 1 l Methylenchlorid bei Zimmertemperatur unter Rühren innerhalb von 60 Minuten zugetropft. Anschliessend kocht man 4 Stunden am Rückfluss und belässt weitere 15 Stunden bei Zimmertemperatur. In die auf 0 °C gekühlte Lösung werden sodann 372 g Kaliumcarbonat in 370 ml Wasser zugetropft, wobei intensiv gerührt wird. Man lässt weitere 2 Stunden bei Zimmertemperatur rühren, dekantiert anschliessend die Methylenchlorid-Phase und extrahiert den Rückstand mit 2 × 500 ml Methylenchlorid.

Die über Kaliumcarbonat getrocknete Methylenchlorid-Phase wird zu einem öligen, teils kristallinen Rückstand eingeengt, sodann in 1 l Hexan aufgekocht und heiss filtriert. Aus dem Filtrat werden nach dem Abdampfen des Hexans 151,4 g des öligen Lactimäthers erhalten : 3-(o-Chlorphenyl)-6-äthoxy-2,3,4,5-tetrahydropyridin (IR(Film) : 1 684 $cm^{-1}$).

Man löst den Lactimäther (151,4 g) in 1,4 l Methanol, gibt 1,3 g wasserfreie p-Toluolsulfonsäure zu und addiert innerhalb von 60 Minuten 85 g 3-Oxo-5-pentensäure-methyl-ester (Nazarov-Reagens). Nach

20 Stunden bei Raumtemperatur werden die leichtflüchtigen Anteile im Vakuum entfernt, der Rückstand anschliessend in Methylenchlorid aufgenommen und mit 500 ml ges. Natriumcarbonat-Lösung gewaschen. Die über Magnesiumsulfat getrocknete Methylenchlorid-Phase wird wiederum eingeengt und der Rückstand aus Essigester-Aether = 4 : 1 (800 ml) kristallisiert. Man erhält 123,7 g (54 %) Methyl-rac-7-(o-chlorphenyl)-3,4,6,7,8,9-hexahydro-2-oxo-2H-chinolizin-1-carboxylat. Smp. 145-147 °C.

Wenn anstelle von 5-(o-Chlorphenyl)-2-piperidon äquimolare Mengen entsprechend substituierter Piperidone verwendet werden, erhält man die folgenden Verbindungen :

Tabelle 5

Methyl-rac-7-($R_a$)-3,4,6,7,8,9-hexahydro-2-oxo-2H-chinolizin-1-carboxylat :

| | Smp. °C |
|---|---|
| $R_a$ = phenyl | 148-149 |
| p-chlorphenyl | 185-187 |
| p-trifluormethylphenyl | 144-145 |
| o-methylphenyl | 183-184 |
| m-methoxyphenyl | 133-134 |
| 2,4-dichlorphenyl | 153-155 |
| 2,6-dichlorphenyl | 159-162 |
| o-fluorphenyl | 165-167 |

Beispiel 2

Eine Lösung von 12,2 g rac-(9aβH)-2α-tert. Butyl-7β-(2,4-dichlorphenyl)octahydro-2H-chinolizin-2-ol in 80 ml Methanol wird mit einer Lösung von 13,3 g (+)-2,2'-(1,1'-Binaphthyl)-phosphorsäure in 250 ml Methanol und 250 ml Methylenchlorid versetzt. Die ausgefallene Gallerte bringt man mit 1,2 l heissem Methanol in Lösung und dampft anschliessend bis auf ein Volumen von 500 ml ein, worauf bei 0 °C das (+)-Phosphat auskristallisiert : 8,7 g (72,5 %), Smp. 288-290 °C. Aus dem (+)-Phosphat kann die (+)-Base [(+) (2R,7S,9aS)-2-tert.Butyl-7-(2,4-dichlorphenyl)octahydro-2H-chinolizin-2-ol] (4,2 g) mit Ammoniak freigesetzt werden, die in 50 ml Aethanol gelöst und mit 2,5 ml 5N Chlorwasserstoff in Aethanol versetzt bei 0 °C 4,0 g (+)-HCl-Salz liefert : Smp. 275-276 °C $[\alpha]_D$ = + 23,1°(c = 1 % in Methanol).

Die Mutterlauge aus der Kristallisation des (+)-Phosphates wird mit konz. Ammoniak basisch gestellt und mit Essigester-Aether = 1 : 1 extrahiert. Die organische Phase wird mit Wasser neutral gewaschen, über Magnesiumsulfat getrocknet und eingedampft : 7,7 g. Diese Base bringt man mit 50 ml Methanol in Lösung und addiert 7,33 g (−)-2,2'-(1,1'-Binaphthyl)-phosphorsäure in 200 ml Methanol und 200 ml Methylenchlorid. Dann wird auf 250 ml eingeengt, 250 ml Aceton addiert und auf 0 °C kalt gestellt. Es kristallisieren 8,2 g (−)-Phosphat aus : Smp. 279-281 °C. Die daraus mit Ammoniak freigesetzte Base [(−) (2S,7R,9aR)-2-tert. Butyl-7-(2,4-dichlorphenyl)octahydro-2H-chinolizin-2-ol] (4,2 g) wird in 50 ml Aethanol gelöst und mit 2,5 ml 5N Chlorwasserstoff in Aethanol versetzt. Bei 0 °C kristallisieren 4,1 g (−)-HCl-Salz aus : Smp. 275-276 °C, $[\alpha]_D$ = − 23,1°(c = 1 % in Methanol).

Analog können die folgenden Enantiomeren durch Spaltung des entsprechenden Racemats isoliert werden :

(+)-2-Butyloctahydro-7-phenyl-2H-chinolizin-2-ol ; Base Smp. 99-101 °C.
(−)-2-Butyloctahydro-7-phenyl-2H-chinolizin-2-ol ; Base Smp. 99-101 °C.

Beispiel 3

Aus 0,54 g Magnesiumspänen in 5 ml abs. Aether und 5 g m-Brombenzotrifluorid in 10 ml Aether wird zunächst das Grignard-Reagens durch 2-stündiges Kochen unter Rückfluss hergestellt. Zu der auf 0 °C abgekühlten Suspension gibt man anschliessend 2,64 g rac-(9aβH)-7β-(p-Chlorphenyl)octahydro-2H-chinolizin-2-on (erhalten gem. Beispiel 1b) in 25 ml abs. Aether tropfenweise zu und rührt eine weitere Stunde bei Raumtemperatur unter Argon.

Zur Hydrolyse wird mit gesättigter Ammoniumchlorid-Lösung versetzt und das Reaktionsprodukt mit Methylenchlorid extrahiert. Die mit gesättigter Natriumchlorid-Lösung gewaschene organische Phase wird über Magnesiumsulfat getrocknet und eingedampft : Ausbeute 4,60 g. Dieser Rückstand ergibt nach Chromatographie an Kieselgel mit Essigester-Methanol 2 diastereomere Produkte :

1,4 g rac-(9aβH)-2α-(m-Trifluormethylphenyl)-7β-(p-chlorophenyl)octahydro-2H-chinolizin-2-ol, Smp. : 119-123 °C (34 %)
1,2 g rac-(9aβH)-2β-(m-Trifluormethylphenyl)-7β-(p-chlorophenyl)octahydro-2H-chinolizin-2-ol, Smp. : 122-124 °C (29 %)

Wenn äquimolare Mengen analoger Alkyl- oder Aryllithium-Reagenzien verwendet werden, können die in Beispiel 1a, Tabelle 2 aufgeführten Verbindungen erhalten werden.

## Beispiel 4

20,0 g rac-(9aβH)-2α-tert. Butyl-7β-(o-chlorphenyl)octahydro-2H-chinolizin-2-ol (erhalten gemäss Beispiel 1a) löst man in 130 ml Isopropenylacetat und addiert 11,7 g wasserfreie p-Toluolsulfonsäure. Darauf wird 1 Stunde unter Rühren am Rückfluss gekocht (Argon). Nach Abkühlen der Reaktionslösung werden noch 20 ml Aether zugegeben. Die Lösung wird 2 Stunden stehen gelassen und dann das ausgefallene p-Toluolsulfonat des Produkts abfiltriert und getrocknet : 22,5 g. Aus dem p-Toluolsulfonat kann die Base (rac-(9aβH)-2α-tert. Butyl-7β-(o-chlorphenyl)octahydro-2H-chinolizin-2-yl-acetat) mit Natriumbicarbonat-Lösung freigesetzt werden : 15,8 g. (70 %). HCl-Salz Smp. 241-242 °C.

Wenn äquimolare Mengen der im Beispiel 1a aufgeführten Alkylcarbinole zur Reaktion eingesetzt werden, können die in Tabelle 6 angegebenen Acetate erhalten werden :

Tabelle 6

rac-(9aβH)-2α-($R_b$)-7β-($R_a$)octahydro-2H-chinolizin-2-yl-acetat :

| | | | Smp. °C |
|---|---|---|---|
| $R_b$ = tert. Butyl | $R_a$ = phenyl | HCl-Salz | 247-248 |
| tert. Butyl | p-chlorphenyl | HCl-Salz | 244-246 |
| tert. Butyl | 2,4-dichlorphenyl | HCl-Salz | 246-247 |
| n-Butyl | phenyl | HCl-Salz | 248-250 |

(+)-(2-Butyloctahydro-7-phenyl-2H-chinolizin-2-yl-acetat). HCl, 242-243 °C.
(−)-(2-Butyloctahydro-7-phenyl-2H-chinolizin-2-yl-acetat). HCl, 242-243 °C.

## Beispiel 5

5,0 g rac-(9aβH)-2α-tert. Butyl-7β-(o-chlorphenyl)octahydro-2H-chinolizin-2-ol werden in 20 ml Pyridin gelöst und innerhalb von 15 Minuten tropfenweise mit 20 ml Trifluoracetanhydrid bei Raumtemperatur versetzt. Nach 22 Stunden bei Raumtemperatur dampft man die Lösung im Vakuum ein, löst den Rückstand in 50 ml Toluol und engt wiederum ein. Anschliessend wird der ölige Rückstand an 100 g Kieselgel mit Essigester chromatographiert. Das Eluat liefert 5,4 g (83,5 %) der Produkt-Base (rac-(9aβH)-2α-tert. Butyl-7β-(o-chlorphenyl)octahydro-2H-chinolizin-2-yl-trifluoracetat). HCl-Salz Smp. 172-173 °C.

Wenn äquimolare Mengen der in den Beispielen 1a und 3 aufgeführten Alkyl- oder Arylcarbinole zur Reaktion verwendet werden, können die in Tabelle 7 aufgeführten Trifluoracetate erhalten werden :

Tabelle 7

rac-(9aβH)-($R_b$)-7β-($R_a$)octahydro-2H-chinolizin-2-yl-trifluoroacetat :

| | | | Smp. °C |
|---|---|---|---|
| $R_b$ = 2α-tert. Butyl | $R_a$ = phenyl | HCl-Salz | 170-172 |
| 2α-phenyl | phenyl | HCl-Salz | 164-166 |

Wenn anstelle von Trifluoracetanhydrid äquimolare Mengen der in den Beispielen 1a und 3 aufgeführten Alkyl- oder Arylcarbinole mit analogen Carbonsäureanhydriden umgesetzt werden, gelangt man zu folgenden Carbinolacylaten :

(Siehe Tabelle 8, Seite 17 f.)

Tabelle 8

rac-(9aβH)-($R_b$)-7β-($R_a$)octahydro-2H-chinolizin-2-yl-($R_c$) :

| | | | | Smp. °C |
|---|---|---|---|---|
| $R_b$ = 2α-phenyl | $R_a$ = o-chlorphenyl | C = acetat | HCl-Salz | 220-222 |
| 2β-phenyl | o-chlorphenyl | acetat | HCl-Salz | 218-220 |
| 2α-phenyl | phenyl | propionat | HCl-Salz | 242-243 |
| 2α-phenyl | m-methoxyphenyl | propionat | HCl-Salz | 212-214 |
| 2β-phenyl | m-methoxyphenyl | propionat | HCl-Salz | 233-235 |
| 2α-m-methoxyphenyl | phenyl | propionat | HCl-Salz | 233-234 |
| 2β-phenyl | phenyl | propionat | Oxalat | 120-122 |

Beispiel 6

a) 3,0 g rac-(9aβH)-2β-phenyl-7β-(o-chlorphenyl)octahydro-2H-chinolizin-2-ol (erhalten gem. Beispiel 1a) löst man in 75 ml Methanol und versetzt innerhalb von 15 Minuten mit 15 ml konz. Schwefelsäure (96 %) und kocht dann 2 Stunden am Rückfluss. Die abgekühlte Reaktionslösung wird auf konz. Natronlauge/Eis gegossen und mit Essigester (3 × 200 ml) extrahiert. Die über Magnesiumsulfat getrocknete organische Phase ergibt nach dem Einengen 3,2 g Rohprodukt, das an 300 g Kieselgel mit Hexan-Aether chromatographiert wird : 1,7 g (54 %) rac-(9aβH)-2β-Methoxy-2-phenyl-7β-(o-chlorphenyl)octahydro-2H-chinolizin. HCl-Salz, Smp. 258-260 °C.

Wenn anstelle von rac-(9aβH)-2β-phenyl-7β-(o-chlorphenyl)octahydro-2H-chinolizin-2-ol entsprechend substituierte Arylcarbinole mit $C_1$-$C_4$-Alkoholen zur Reaktion gebracht werden, erhält man folgende O-($C_1$-$C_4$)Alkyl-Verbindungen :

Tabelle 9

rac-(9aβH)-2β-($R_b$)-2-phenyl-7β-($R_a$)octahydro-2H-chinolizin :

| | | | Smp. °C |
|---|---|---|---|
| $R_b$ = methoxy | $R_a$ = phenyl | HCl-Salz | 277-279 |
| aethoxy | phenyl | HCl-Salz | 254-255 |

b) 0,5 g rac-(9aβH)-2α-phenyl-7β-(o-chlorphenyl)octahydro-2H-chinolizin-2-ol (erhalten gem. Beispiel 1a) werden in 5 ml HMPA (Hexamethyl-phosphorsäuretriamid) gelöst und bei 0 °C mit 0,1 g Natriumhydrid (50-60 % in Mineralöl) versetzt. Man rührt 1/2 Stunde bei 0 °C und fügt dann 0,2 ml Methyljodid zu. Anschliessend wird 15 Stunden bei Zimmertemperatur gerührt. Zur Aufarbeitung giesst man auf 50 ml Wasser und extrahiert 2 × mit 50 ml Essigester. Die Essigesterphase wird zusätzlich 3 × mit je 50 ml Wasser gewaschen, anschliessend über Magnesiumsulfat getrocknet und eingedampft. Chromatographie an 50 g Kieselgel mit Hexan-Aether ergibt 0,2 g (38 %) rac-(9aβH)-2β-Methoxy-2-phenyl-7β-(o-chlorphenyl)octahydro-2H-chinolizin. HCl-Salz, Smp. 258-260 °C.

Beispiel 7

a) Eine Lösung von 7,45 g rac-1-(7β-)-(o-Chlorphenyl)-3,6,7,8,9,9aβ-hexahydro-4H-chinolizin-2-yl)-5,6-dimethyl-2-benzimidazolinon in 30 ml konz. Schwefelsäure und 300 ml abs. Aethanol wird mit 0,40 g Platinoxid während 6 Stunden bei Raumtemperatur hydriert. Anschliessend filtriert man vom Katalysator ab, dampft das Filtrat ein und verteilt den Rückstand zwischen Chloroform und 2N Natronlauge. Die über Kaliumcarbonat getrocknete Chloroformphase ergibt nach dem Eindampfen 7,5 g Rohprodukt. Zur Reinigung wird das HCl-Salz aus Methanol-Aether kristallisiert : 6,7 g (90 %) rac-1-((9aβH)-7β-(o-Chlorphenyl)octahydro-2H-chinolizin-2α-yl)-5,6-dimethyl-2-benzimidazolinon-hydrochlorid, Smp. : 310 °C. Als Variante zu diesem Reinigungsverfahren kann die Base an Kieselgel mit Methylenchlorid-Methanol chromatographiert werden. Smp. Base : 268-272 °C.

Wenn anstelle von rac-1-(7β-(o-Chlorphenyl)-3,6,7,8,9,9aβ-hexahydro-4H-chinolizin-2α-yl)-5,6-dimethyl-2-benzimidazolinon äquimolare Mengen entsprechend substituierter Edukte mit den in Tabelle 10 angegebenen Katalysatoren hydriert werden, gelangt man zu folgenden Produkten :

(Siehe Tabelle 10, Seite 18 f.)

Tabelle 10

rac-1-((9aβH)-7β-($R_a$)octahydro-2H-chinolizin-2α-yl)-(B)-2-benzimidazolinon :

| | | Katalysator : | | Smp. °C |
|---|---|---|---|---|
| $R_a$ = o-Chlorphenyl | B = 5,6-dichlor | $PtO_2$ | HCl-Salz | 300-305 |
| o-Chlorphenyl | 5,6-H | $PtO_2$ | HCl-Salz | 315-318 |
| o-Chlorphenyl | 5,6-dimethoxy | $PtO_2$ | HCl-Salz | 229-235 |
| o-Chlorphenyl | 5-$(CH_2)_4$-6 | $PtO_2$ | HCl-Salz | 245-250 |
| p-Chlorphenyl | 5,6-H | $PtO_2$ | Base | 244-248 |
| p-Chlorphenyl | 5,6-dimethyl | $PtO_2$ | Base | 289-295 |
| o-Methylphenyl | 5,6-H | 10 % Pd/C | HCl-Salz | 285-290 |
| o-Methylphenyl | 5,6-dimethyl | 10 % Pd/C | HCl-Salz | 291-293 |
| o-Methylphenyl | 5,6-dichlor | $PtO_2$ | Base | 294-297 |
| Phenyl | 5,6-H | 10 % Pd/C | HCl-Salz | 223-228 |
| Phenyl | 5,6-dimethyl | 10 % Pd/C | HCl-Salz | 295-302 |
| Phenyl | 5,6-dichlor | $PtO_2$ | Base | 295-300 |
| Phenyl | 5,6-dimethoxy | 10 % Pd/C | Base | 248-250 |
| o-Fluorphenyl | 5,6-dimethyl | 10 % Pd/C | HCl-Salz | > 300 °C |
| o-Fluorphenyl | 5,6-dichlor | $PtO_2$ | HCl-Salz | > 250 °C |
| o-Fluorphenyl | 5,6-difluor | $PtO_2$ | HCl-Salz | > 300 °C |
| o-Fluorphenyl | 5-$(CH_2)_4$-6 | $PtO_2$ | HCl-Salz | 248-250 °C |

b) Das als Ausgangsmaterial verwendete rac-1-(7β-(o-Chlorphenyl)-3,6,7,8,9,9aβ-hexahydro-4H-chinolizin-2-yl)-5,6-dimethyl-2-benzimidazolinon kann wie folgt erhalten werden :

6,35 g 4,5-Dimethyl-1,2-phenylendiamin löst man in 100 ml Xylol, erwärmt auf Rückflusstemperatur und addiert innerhalb von 2 Stunden 15 g Methyl-rac-(1αH,9aβH)-7β-(o-chlorphenyl)octahydro-2-oxo-2H-chinolizin-1-carboxylat (erhalten gem. Beispiel 1c) in 100 ml Xylol. Danach kocht man 7 Stunden unter Rückfluss und kühlt dann ab. Aus der Reaktionslösung kristallisieren 13,65 g rac-1(7β-(o-Chlorphenyl)-3,6,7,8,9,9aβ-hexahydro-4H-chinolizin-2-yl)-5,6-dimethyl-2-benzimidazolinon aus. (72 %) Smp. 239-241 °C.

Wenn anstelle von Methyl-rac-(1αH,9aβH)-7β-(o-chlorphenyl)octahydro-2-oxo-2H-chinolizin-1-carboxylat äquimolare Mengen entsprechend substituierter β-Ketoester mit 1,2-Phenylendiaminen umgesetzt werden, gelangt man zu folgenden Produkten :

Tabelle 11

rac-1-(7β-($R_a$)-3,6,7,8,9-aβ-hexahydro-4H-chinolizin-2-yl)-(B)-2-benzimidazolinon :

| | | | Smp. °C |
|---|---|---|---|
| $R_a$ = o-Chlorphenyl | B = 5,6-dichlor | HCl-Salz | 281-284 |
| o-Chlorphenyl | 5,6-H | HCl-Salz | 267-269 |
| o-Chlorphenyl | 5,6-dimethoxy | HCl-Salz | 225 (Zers.) |
| o-Chlorphenyl | 5-$(CH_2)_4$-6 | Base | 246-249 |
| p-Chlorphenyl | 5,6-H | HCl-Salz | 274-277 |
| p-Chlorphenyl | 5,6-dimethyl | HCl-Salz | 228-230 |
| o-Methylphenyl | 5,6-H | HCl-Salz | 277-279 |
| o-Methylphenyl | 5,6-dimethyl | HCl-Salz | 226-230 |
| o-Methylphenyl | 5,6-dichlor | HCl-Salz | 286-289 |
| phenyl | 5,6-dimethyl | Base | 259-262 |
| phenyl | 5,6-dichlor | Base | 293-296 |
| phenyl | 5,6-dimethoxy | Base | 241-243 |
| 2'-Methoxy-5'-chlor-phenyl | 5,6-dimethyl | Base | 248-251 |
| o-Fluorphenyl | 5,6-dimethyl | Base | 260-263 |
| o-Fluorphenyl | 5,6-dichlor | Base | 298-302 |
| o-Fluorphenyl | 5,6-difluor | Base | 257-259 |
| o-Fluorphenyl | 5-$(CH_2)_4$-6 | Base | 238-241 |

## Beispiel 8

3,0 g rac-1-((9aβH)-7β-(o-Chlorphenyl)octahydro-2H-chinolizin-2α-yl)-2-benzimidazolinon (erhalten

gem. Beispiel 7a) werden in 30 ml trockenem Monoglym suspendiert und mit 0,41 g Natriumhydrid (50-60 % in Mineralöl) versetzt und 1 Stunde bei Raumtemperatur gerührt. Anschliessend kühlt man auf 0 °C und addiert 1,23 g Methyljodid. Nach 30 Minuten bei Raumtemperatur wird auf 50 ml Wasser gegossen und mit Essigester extrahiert. Die über Magnesiumsulfat getrocknete organische Phase wird eingeengt und das Rohprodukt an Kieselgel mit Methylenchlorid-Methanol chromatographiert. Es resultieren 2,10 g (58 %) rac-1-((9aβH)-7β-(o-Chlorphenyl)octahydro-2H-chinolizin-2α-yl)-3-methyl-2-benzimidazolinon. HCl-Salz, Smp. 315-320 °C.

## Beispiel 9

0,5 g rac-1-((9aβH)-7β-(o-Chlorphenyl)octahydro-2H-chinolizin-2α-yl)-5,6-dimethyl-2-benzimidazolinon (erhalten gem. Beispiel 7a) werden mit 1,0 g Phosphorpentasulfid in 5 ml Pyridin 3 Stunden auf 150 °C erwärmt. Anschliessend dampft man das Reaktionsgemisch ein und verteilt den Rückstand zwischen 2N Natronlauge und Chloroform-Isopropanol. Die organische Phase wird mit gesättigter Natriumchlorid-Lösung gewaschen, über Magnesiumsulfat getrocknet und eingedampft. Chromatographie des Rohprodukts an Kieselgel mit Methylenchlorid-Methanol ergibt 0,22 g rac-1-((9aβH)-7β-(o-Chlorphenyl)octahydro-2H-chinolizin-2α-yl)-5,6-dimethyl-2-benzimidazolinthion. Smp. 293-297 °C.

## Beispiel 10

a) Eine Lösung 7,20 g rac-(9aβH)-7β-(o-Fluorphenyl)octahydro-2α-(α′,α′,α′-trifluor-2-amino-p-toluidino)-2H-chinolizin in 180 ml Methylenchlorid wird mit 4,59 g N,N′-Carbonyl-diimidazol (93 %ig) versetzt und über Nacht bei Raumtemperatur gerührt. Das ausgefallene Reaktionsprodukt wird nach 20 Stunden abfiltriert, mit 3 × 100 ml Methylenchlorid gewaschen und anschliessend getrocknet. Man erhält 6,45 g (83,5 %) kristallines rac-1-[(9aβH)-7β-(o-Fluorphenyl)octahydro-2H-chinolizin-2α-yl]-5-(trifluormethyl)-2-benzimidazolinon. Smp. 304-306 °C.

Wenn anstelle von rac-(9aβH)-7β-(o-Fluorphenyl)octahydro-2α-(α′,α′,α′-trifluor-2-amino-p-toluidino)-2H-chinolizin entsprechend substituierte Phenylendiamine mit N,N′-Carbonyl-diimidazol umgesetzt werden, gelangt man zu folgenden Produkten :

Tabelle 12

rac-1-[(9aβH)-7β-($R_a$)octahydro-2H-chinolizin-2α-yl]-(B)-2-benzimidazolinon :

| | | Smp. °C | |
|---|---|---|---|
| $R_a$ = o-Fluorphenyl | B = 5-chlor | 283-285 | (Base) ; 298-304 (HCl-Salz) |
| o-Chlorphenyl | 5-chlor | 223-230 | (Base) |
| o-Chlorphenyl | 5-trifluormethyl | 257-259 | (Base) ; 240-245 (HCl-Salz) |
| o-Chlorphenyl | 5,6-dichlor | 300-305 | (HCl-Salz) |
| o-Fluorphenyl | 5,6-dichlor | > 250 | (HCl-Salz) |

b) Das als Ausgangsmaterial in Beispiel 10a verwendete rac-(9aβH)-7β-(o-Fluorphenyl)octahydro-2α-(α′,α′,α′-trifluor-2-amino-p-toluidino)-2H-chinolizin kann wie folgt erhalten werden :

25,7 g rac-(9aβH)-7β-(o-Fluorphenyl)octahydro-2α-(α′,α′,α′-trifluor-2-nitro-p-toluidino)-2H-chinolizin werden in 0,9 l Tetrahydrofuran und 0,9 l Methanol mit 13 g Raney-Nickel während 20 Stunden bei Raumtemperatur und Atmosphärendruck hydriert. Anschliessend saugt man vom Katalysator ab und dampft das Filtrat ein. Es resultieren 23,6 g (99 %) kristallines rac-(9aβH)-7β-(o-Fluorphenyl)-octahydro-2α-(α′,α′,α′-trifluor-2-amino-p-toluidino)-2H-chinolizin, das gegebenenfalls aus Alkohol-Essigester umkristallisiert werden kann. Smp. 133-138 °C.

Werden anstelle von rac-(9aβH)-7β-(o-Fluorphenyl)octahydro-2α-(α′,α′,α′-trifluor-2-nitro-p-toluidino)-2H-chinolizin entsprechend substituierte Nitroverbindungen reduziert, so gelangt man zu folgenden Produkten :

Tabelle 13

rac-(9aβH)-7β-($R_a$)octahydro-2α-(B)-2H-chinolizin :

| | | Smp. °C |
|---|---|---|
| $R_a$ = o-Fluorphenyl | B = 2-amino-4-chlor-anilino | 137-141 |
| o-Fluorphenyl | 2-amino-4,5-dichlor-anilino | 164-166 |
| o-Chlorphenyl | 2-amino-4-chlor-anilino | 170-172 |
| o-Chlorphenyl | 2-amino-4,5-dichlor-anilino | 148-150 |
| o-Chlorphenyl | α′,α′,α′-trifluor-2-amino-p-toluidino | 151-153 |

c) Das als Ausgangsmaterial in Beispiel 10b eingesetzte rac-(9aβH)-7β-(o-Fluorphenyl)octahydro-2α-(α',α',α'-trifluor-2-nitro-p-toluidino)-2H-chinolizin kann wie folgt erhalten werden :

20,0 g rac-(9aβH)-7β-(o-Fluorphenyl)-2α-(amino)octahydro-2H-chinolizin werden in 300 ml Cyclohexanol (99 %) gelöst, mit 8,62 g Natriumcarbonat (wasserfrei) versetzt und auf 160 °C erwärmt. Bei dieser Temperatur tropft man innerhalb von 3 Stunden eine Lösung von 20,0 g 4-Chlor-3-nitro-benzotrifluorid in 200 ml Cyclohexanol (> 99 %) zu. Anschliessend wird die Reaktionslösung für weitere 4 Stunden bei 160 °C gehalten. Nach Abkühlen auf Raumtemperatur wird vom anorganischen Rückstand abfiltriert und das Filtrat im Vakuum eingedampft. Den Abdampfrückstand löst man in 500 ml heissem Essigester, setzt 5 g Aktivkohle zu und kocht 5 Minuten am Rückfluss. Danach wird heiss durch Hyflo Super Cel filtriert, das Filtrat auf 200 ml eingeengt und 100 ml n-Hexan zugesetzt. Es kristallisieren 22,3 g rac-(9aβH)-7β-(o-Fluorphenyl)octahydro-2α-(α',α',α'-trifluor-2-nitro-p-toluidino)-2H-chinolizin. Smp. 163-165 °C. Durch Chromatographie der Mutterlauge an 250 g Kieselgel mit n-Hexan/Essigester = 4 : 1 werden nochmals 5,45 g Produkt erhalten. Gesamtausbeute 78,8 %.

Falls anstelle von rac-(9aβH)-7β-(o-Fluorphenyl)-2α-(amino)octahydro-2H-chinolizin entsprechend substituierte Amino-chinolizidine mit entsprechend substituierten o-Chlor-nitrobenzolen umgesetzt werden, so erhält man folgende Produkte :

Tabelle 14

rac-(9aβH)-7β-($R_a$)octahydro-2α-(B)-2H-chinolizin :

| | | Smp. °C |
|---|---|---|
| $R_a$ = o-Fluorphenyl | B = 2-nitro-4-chlor-anilino | 185-187 |
| o-Fluorphenyl | 2-nitro-4,5-dichlor-anilino | 202-203 |
| o-Chlorphenyl | 2-nitro-4-chlor-anilino | 183-185 |
| o-Chlorphenyl | 2-nitro-4,5-dichlor-anilino | 197-199 |
| o-Chlorphenyl | α',α',α'-trifluor-2-nitro-p-toluidino | 154-155 |

d) Das als Ausgangsmaterial in Beispiel 10c) eingesetzte rac-(9aβH)-7β-(o-Fluorphenyl)-2α-(amino)octahydro-2H-chinolizin kann wie folgt erhalten werden :

84,3 g rac-(9aβH)-7β-(o-Fluorphenyl)octahydro-2H-chinolizin-2-on (vgl. Beispiel 1b) Tabelle 3) werden in 1 700 ml Methanol mit 47,4 g Hydroxylamin-Hydrochlorid und 94,3 g Kaliumcarbonat (wasserfrei) während 1 1/2 Stunden auf Rückfluss erhitzt. Anschliessend destilliert man das Methanol ab und verteilt den Rückstand zwischen 750 ml Wasser, 600 ml Chloroform und 150 ml Isopropanol. Die wässrige Phase wird zweimal mit je 250 ml eines Gemisches aus 200 ml Chloroform und 50 ml Isopropanol nachextrahiert, die gesammelten organischen Phasen über Magnesiumsulfat getrocknet und anschliessend eingedampft. Es resultieren 85,3 g Oxim, welches in 1,5 l Tetrahydrofuran, 0,5 l Alkohol sowie 1 l (5 % g/g Ammoniak in Alkohol) gelöst und mit 45 g Raney-Nickel während 30 Stunden hydriert wird (Raumtemperatur, Atmosphärendruck). Danach wird vom Katalysator abfiltriert und das Filtrat eingedampft. Aus dem Rückstand können durch Lösen in 400 ml Alkohol und Addieren von 130 ml 5N Chlorwasserstoff in Alkohol 53,5 g des Dihydrochlorid-Salzes von rac-(9aβH)-7β-(o-Fluorphenyl)-2α-(amino)octahydro-2H-chinolizin auskristallisiert werden. Smp. 299-302 °C. Das diastereomere rac-(9aβH)-7β-(o-Fluorphenyl)-2β-(amino)octahydro-2H-chinolizin ist als Dihydrochlorid in der Mutterlauge gelöst.

Wenn anstelle von rac-(9aβH)-7β-(o-Fluorphenyl)octahydro-2H-chinolizin-2-on entsprechend substituierte Chinolizidinone aminiert werden, so erhält man folgende Produkte :

Tabelle 15

rac-(9aβH)-7β-($R_a$)-2α-(amino)octahydro-2H-chinolizin :

$R_a$ = o-Chlorphenyl Smp. °C (2HCl) : 293-296 °C

## Beispiel 11

Eine Lösung von 7,20 g rac-(9aβH)-7β-(o-Fluorphenyl)-octahydro-2α-(α',α',α'-trifluor-2-amino-p-toluidino)-2H-chinolizin (Beispiel 10b) in 180 ml Methylenchlorid wird mit 4,75 g N,N'-Thiocarbonyl-diimidazol versetzt und über Nacht bei Zimmertemperatur gerührt. Nach 20 Stunden wird das ausgefallene Reaktionsprodukt abfiltriert, mit 2 × 100 ml Methylenchlorid gewaschen und anschliessend getrocknet. Man erhält 5,80 g (73,0 %) kristallines rac-1-[(9aβH)-7β-(o-Fluorphenyl)octahydro-2H-chinolizin-2α-yl]-5-(trifluormethyl)-2-benzimidazolinthion. Smp. 308-309 °C.

Wenn anstelle von rac-(9aβH)-7β-(o-Fluorphenyl)octahydro-2α-(α',α',α'-trifluor-2-amino-p-toluidino)-2H-chinolizin entsprechend substituierte Phenylendiamine mit N,N'-Thiocarbonyl-diimidazol umgesetzt werden, gelangt man zu folgenden Produkten :

Tabelle 16

rac-1-[(9aβH)-7β-($R_a$)octahydro-2H-chinolizin-2α-yl]-(B)-2-benzimidazolinthion :

| | | Smp. °C |
|---|---|---|
| $R_a$ = o-Fluorphenyl | B = 5-chlor | > 300 |
| o-Fluorphenyl | 5,6-dichlor | > 310 |
| o-Chlorphenyl | 5-chlor | 264-267 |
| o-Chlorphenyl | 5,6-dichlor | > 295 |

Beispiel 12

Eine Lösung von 5,80 g rac-(9aβH)-2α-(2-Amino-4,5-dichlor-anilino)-7β-(o-chlorphenyl)octahydro-2H-chinolizin (Beispiel 10b) in 60 ml m-Xylol werden mit 20 ml Orthoessigsäuretriäthylester während 6 Stunden am Rückfluss gekocht. Nach Abkühlen der Reaktionslösung giesst man auf kalte 2N Natronlauge und extrahiert 2 mal mit je 300 ml Methylenchlorid. Die über Natriumcarbonat (wasserfrei) getrocknete organische Phase wird anschliessend eingedampft. Den Rückstand löst man in 50 ml 5N Salzsäure in Alkohol und erwärmt während 1 Stunde auf Rückfluss. Das ausgefallene Dihydrochlorid des Reaktions produkts wird abfiltriert und getrocknet. Es resultieren 5,60 g des Dihydrochlorid-Salzes von rac-(9aβH)-7β-(o-Chlorphenyl)-2α-(5,6-dichlor-2-methyl-1-benzimidazolyl)octahydro-2H-chinolizin. Smp. 231-236 °C. Ausbeute 77 %.

Falls anstelle von rac-(9aβH)-2α-(2-Amino-4,5-dichloranilino)-7β-(o-chlorphenyl)octahydro-2H-chinolizin entsprechend substituierte Phenylendiamine mit Orthoessigsäuretriäthylester umgesetzt werden, können folgende Produkte erhalten werden :

Tabelle 17

rac-(9aβH)-7β-($R_a$)-2α-((B)-2-methyl-1-benzimidazolyl)octahydro-2H-chinolizin. 2 HCl :

| | | Smp. °C |
|---|---|---|
| $R_a$ = o-Chlorphenyl | B = 5-chlor | 240-245 |

Beispiel 13

2,70 g eines (1 : 1)-Gemisches von rac-(9aβH)-2-Phenyl-7β-(o-chlorphenyl)-3,4,6,7,8,9-hexahydro-2H-chinolizin und rac-(9aβH)-2-Phenyl-7β-(o-chlorphenyl)-1,4,6,7,8,9-hexahydro-2H-chinolizin werden mit 70 ml Wasser und 14 ml Schwefelsäure (98 %) 15 Stunden unter Rückfluss gekocht. Nach Abkühlen der Reaktionslösung wird auf gesättigte Kaliumcarbonat/Wasser-Lösung gegossen, mit 200 ml Wasser verdünnt und dreimal mit je 200 ml Essigester extrahiert. Die vereinigten Essigester-Extrakte trocknet man über Magnesiumsulfat und dampft ein. Das Rohprodukt (2,35 g) chromatographiert man über 100 g Kieselgel mit Essigester als Eluens. In einer ersten Fraktion werden 1,55 g Eduktgemisch zurückgewonnen ; aus einer weiteren Fraktion können 0,15 g (Ausbeute 3,5 %) rac-(9aβH)-2α-Phenyl-7β-(o-chlorphenyl)octahydro-2H-chinolizin-2-ol erhalten werden. Smp. (HCl-Salz) : 265-275 °C.

Das als Ausgangsmaterial eingesetzte Doppelbindungsisomerengemisch kann wie folgt erhalten werden :

5,00 g rac-(9aβH)-2β-Phenyl-7β-(o-chlorophenyl)octahydro-2H-quinolizin-2-ol (Beispiel 1a, Tabelle 2) löst man in 25 ml Pyridin und addiert 2,1 ml Thionylchlorid. Nach zweistündigem Rühren bei Zimmertemperatur wird im Vakuum eingedampft und anschliessend zwischen Chloroform und Wasser verteilt. Die über Magnesiumsulfat getrocknete organische Phase dampft man ein und filtriert das Rohprodukt (3,90 g) mit Essigester über Aluminiumoxid (200 g, Aktivität III). Auf diese Weise können 2,75 g eines (1 : 1)-Gemisches von rac-(9aβH)-2-Phenyl-7β-(o-chlorphenyl)-3,4,6,7,8,9-hexahydro-2H-chinolizin und rac-(9aβH)-2-Phenyl-7β-(o-chlorphenyl)-1,4,6,7,8,9-hexahydro-2H-chinolizin eluiert werden. NMR($CDCl_3$) : 5,93 + 6,08 ppm (je 2 breite Singuletts im Verhältnis 1 : 1, zusammen 1 Proton).

Beispiel 14

0,068 g Natriumhydrid (50 % in Mineralöl) in 10 ml 1,2-Dimethoxyäthan suspendiert, werden mit 0,160 g Benzimidazolinon und anschliessend mit 0,500 g eines (1 : 1)-Gemisches von rac-(9aβH)-7β-(o-Chlorphenyl)octahydro-2H-chinolizin-2α-yl-p-toluolsulfonat und rac-(9aβH)-7β-(o-Chlorphenyl)octahydro-2H-chinolizin-2β-yl-p-toluolsulfonat in 10 ml 1,2-Dimethoxyäthan versetzt und anschliessend 45

Stunden auf Rückfluss erhitzt. Nach Abkühlen verteilt man zwischen Chloroform und Wasser, trocknet die organische Phase über Magnesiumsulfat und dampft ein. Das erhaltene Rohprodukt (0,450 g) chromatographiert man an 50 g Kieselgel mit Chloroform (1 % Methanol) als Eluens und erhält 0,040 g (Ausbeute 9 %) rac-1-((9aβH)-7β-(o-Chlorphenyl)octahydro-2H-chinolizin-2α-yl)-2-benzimidazolinone. Smp. (HCl-Salz) : 315-317 °C.

Das als Ausgangsmaterial verwendete (1 : 1)-Gemisch diastereomerer p-Toluolsulfonate kann wie folgt hergestellt werden :

5,00 g rac-(9aβH)-7β-(o-Chlorphenyl)octahydro-2H-chinolizin-2-on (Beispiel 1b) in 50 ml Methanol werden mit 0,7 g Natriumborhydrid versetzt und 45 Minuten bei Zimmertemperatur gerührt. Anschliessend verteilt man zwischen Wasser und Essigester, trocknet die organische Phase über Magnesiumsulfat und engt ein. Das erhaltene Rohprodukt wird über 200 g Kieselgel mit Diäthyläther chromatographiert und das Eluat eingedampft : 5,0 g als Gemisch diastereomerer Alkohole. Das Gemisch der Alkohole löst man in 50 ml Pyridin und setzt 7,3 g p-Toluolsulfonsäurechlorid zu. Nach zweistündigem Rühren bei Zimmertemperatur wird eingedampft, der Rückstand zwischen 2-n Natronlauge und Essigester verteilt und nach Trocknen der organischen Phase eingeengt. Durch Chromatographie an 100 g Kieselgel mit Methylenchlorid (1 % Methanol) können 2,20 g eines (1 : 1)-Gemisches von rac-(9aβH)-7β-(o-Chlorphenyl)-octahydro-2H-chinolizin-2β-yl-p-toluolsulfonat und rac-(9aβH)-7β-(o-Chlorphenyl)-octahydro-2H-chinolizin-2α-yl-p-toluolsulfonat (NMR($CDCl_3$) : 2 Singuletts 2,33 + 2,43 ppm) sowie 5,80 g rac-(9aβH)-7β-(o-Chlorphenyl)-octahydro-2H-chinolizin-2α-yl-p-toluolsulfonat (NMR($CDCl_3$) : 1 Singulett 2,43 ppm) erhalten werden.

**Ansprüche**

1. Phenylchinolizine der allgemeinen Formel

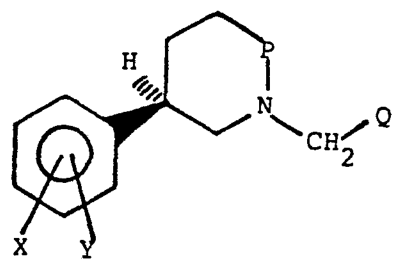

in der X Wasserstoff, Fluor, Chlor, $C_{1-6}$-Alkoxy, $C_{1-6}$-Alkyl oder Trifluormethyl und Y Wasserstoff, Fluor, Chlor, $C_{1-6}$-Alkoxy oder $C_{1-6}$-Alkyl bedeuten und P und Q zusammen eine der Gruppen

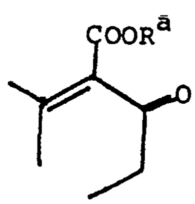 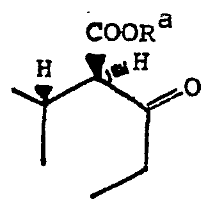 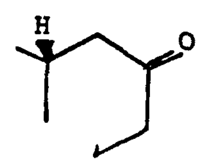 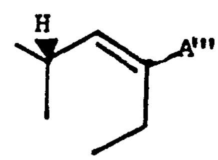

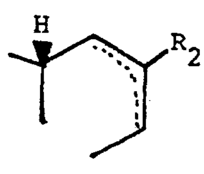 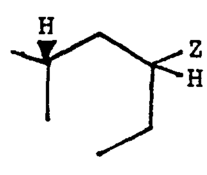 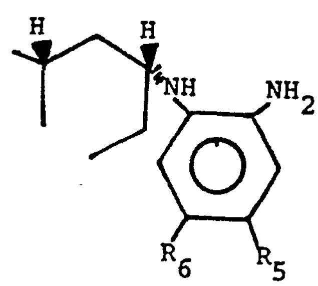

darstellt, worin $R^a$ $C_1$-$C_6$-Alkyl, $R^2$ $C_{1-6}$-Alkyl, gegebenenfalls durch Halogen, $C_{1-6}$-Alkoxy oder Trifluormethyl substituiertes Phenyl, $R^5$ und $R^6$ unabhängig voneinander Wasserstoff, Methyl, Fluor, Chlor, Methoxy, Trifluormethyl oder zusammen $-(CH_2)_4-$, Z eine abspaltbare Gruppe und A''' eine Gruppe mit der Bedeutung

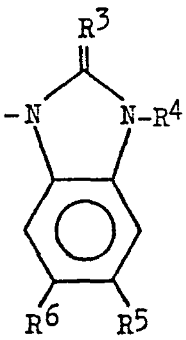

oder

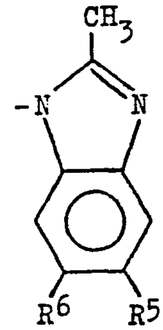

mit folgender Bedeutung für $R^3$-$R^6$ :

$R^3$ : Sauerstoff oder Schwefel,

$R^4$ : Wasserstoff oder $C_{1-6}$-Alkyl,

$R^5$ und $R^6$ unabhängig voneinander : Wasserstoff, Methyl, Fluor, Chlor, Methoxy, Trifluormethyl oder zusammen $—(CH_2)_4$, wobei falls $R^4$ $C_1$-$C_6$-Alkyl bedeutet, $R^3$ Sauerstoff darstellt, bedeuten, in Form des Racemats oder der Enantiomeren.

2. Phenylchinolizine gemäss Anspruch 1 der allgemeinen Formel

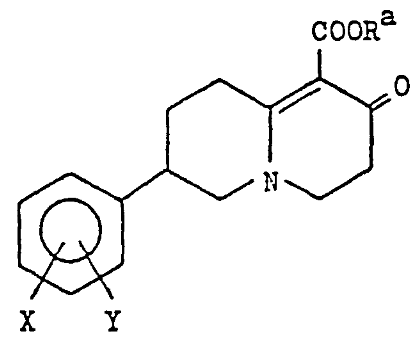

(XIII)

worin X, Y und $R^a$ dasselbe wie in Anspruch 1 bedeuten.

3. Phenylchinolizine gemäss Anspruch 1 der allgemeinen Formel

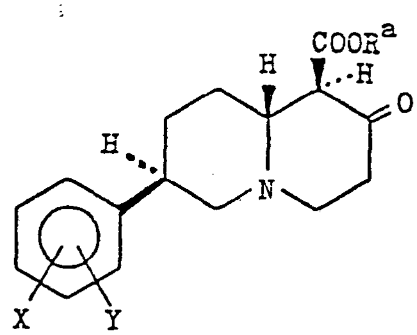

(XIV)

worin X, Y und $R^a$ dasselbe wie in Anspruch 1 bedeuten in Form des Racemats oder der Enantiomeren.

4. Phenylchinolizine gemäss Anspruch 1 der allgemeinen Formel

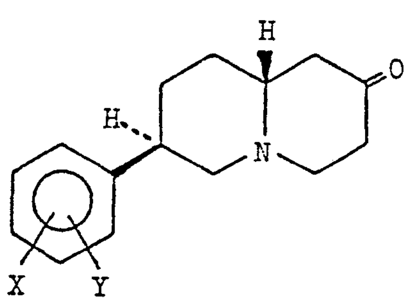

(V)

worin X und Y dasselbe wie in Anspruch 1 bedeuten in Form des Racemats oder der Enantiomeren.

5. Phenylchinolizine gemäss Anspruch 1 der allgemeinen Formel

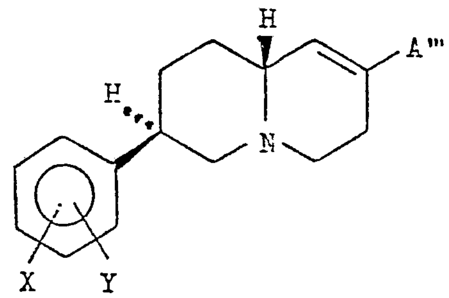

(IX) + (XV)

worin X, Y und $A'''$ dasselbe wie in Anspruch 1 bedeuten, in Form des Racemats oder der Enantiomeren.

6. Phenylchinolizine gemäss Anspruch 1 der allgemeinen Formel

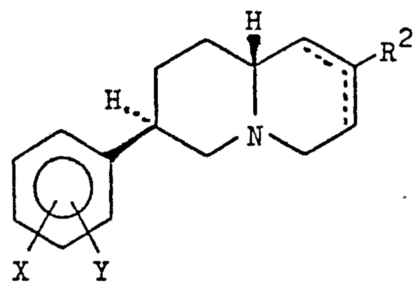

(VI)

worin X, Y und $R^2$ dasselbe wie in Anspruch 1 bedeuten und die gestrichelte Bindung eine Doppelbindung in 1,2- oder 2,3-Stellung darstellt, in Form des Racemats oder der Enantiomeren.

7. Phenylchinolizine gemäss Anspruch 1 der allgemeinen Formel

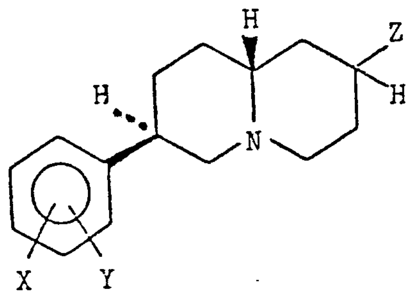

(VII)

worin X, Y und Z dasselbe wie in Anspruch 1 bedeuten, in Form des Racemats oder der Enantiomeren.

8. Phenylchinolizine gemäss Anspruch 1 der allgemeinen Formel

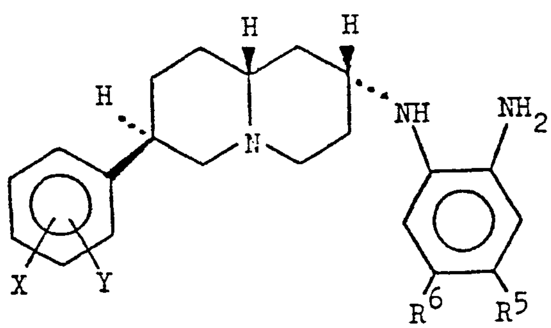

(X)

worin X, Y, $R^5$ und $R^6$ dasselbe wie in Anspruch 1 bedeuten, in Form des Racemats oder der Enantiomeren.

9. Eine Verbindung gemäss Anspruch 4, worin X und Y Wasserstoff darstellen.

10. Eine Verbindung gemäss Anspruch 2, worin X und Y Wasserstoff darstellen.

11. Eine Verbindung gemäss Anspruch 3, worin X und Y Wasserstoff darstellen.

12. Rac-(9αβH)-7β-(o-Fluorphenyl)octahydro-2α-(α',α',α'-trifluor-2-amino-p-toluidino)-2H-chinolizin.

**Claims**

1. Phenylquinolizines of the general formula

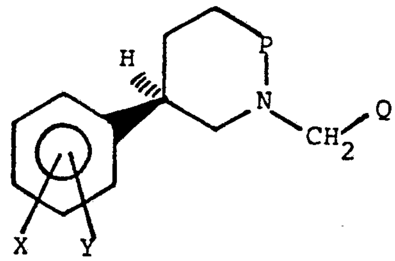

(I)

in which X signifies hydrogen, fluorine, chlorine, $C_{1-6}$-alkoxy, $C_{1-6}$-alkyl or trifluoromethyl and Y signifies hydrogen, fluorine, chlorine, $C_{1-6}$-alkoxy or $C_{1-6}$-alkyl and P and Q together represent one of the groups

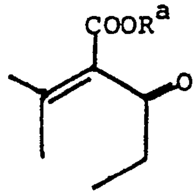
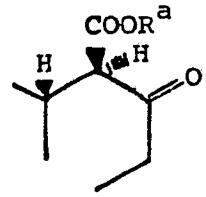
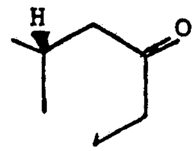
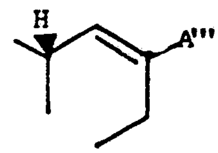

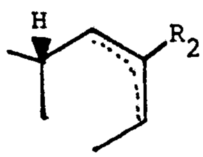
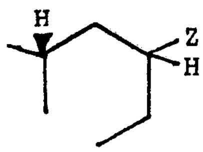
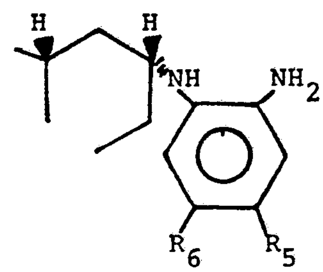

wherein $R^a$ signifies $C_1$-$C_6$-alkyl, $R^2$ signifies $C_{1-6}$-alkyl, phenyl optionally substituted by halogen, $C_{1-6}$-alkoxy or trifluoromethyl, $R^5$ and $R^6$ independently of one another signify hydrogen, methyl, fluorine, chlorine, methoxy, trifluoromethyl or together signify $-(CH_2)_4-$, Z signifies a cleavable group and A''' signifies a group with the significance

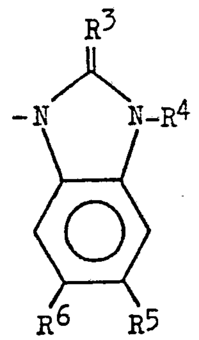

or

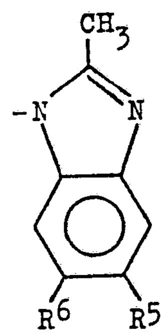

with the following significances for $R^3$-$R^6$ :

$R^3$ : oxygen or sulphur,

$R^4$ : hydrogen or $C_{1-6}$-alkyl,

$R^5$ and $R^6$ independently of one another: hydrogen, methyl, fluorine, chlorine, methoxy, trifluoromethyl or together $-(CH_2)_4$, $R^4$ signifying $C_1$-$C_6$-alkyl when $R^3$ represents oxygen, in the form of the racemate or of the enantiomers.

2. Phenylquinolizines according to claim 1 of the general formula

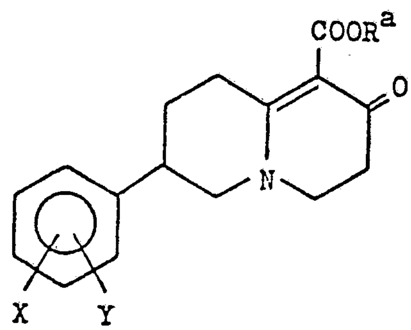

(XIII)

wherein X, Y and $R^a$ signify the same as in claim 1.

3. Phenylquinolizines according to claim 1 of the general formula

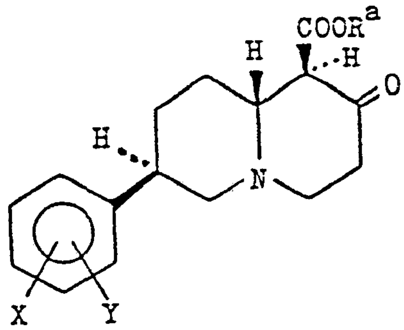

(XIV)

wherein X, Y and $R^a$ signify the same as in claim 1, in the form of the racemate or of the enantiomers.

4. Phenylquinolizines according to claim 1 of the general formula

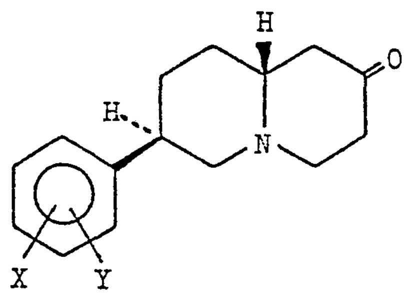

(V)

wherein X and Y signify the same as in claim 1, in the form of the racemate or of the enantiomers.

5. Phenylquinolizines according to claim 1 of the general formula

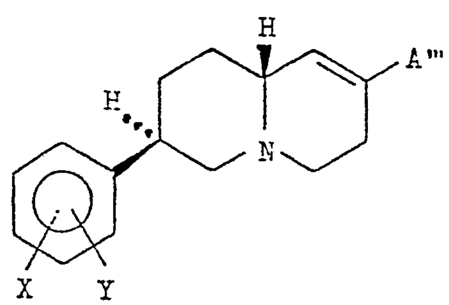

(IX) + (XV)

wherein X, Y and A''' signify the same as in claim 1, in the form of the racemate or of the enantiomers.

6. Phenylquinolizines according to claim 1 of the general formula

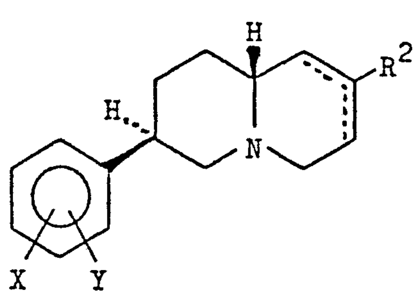

(VI)

wherein X, Y and $R^2$ signify the same as in claim 1 and the dotted bond represents a double bond in the 1,2- or 2,3-position, in the form of the racemate or of the enantiomers.

7. Phenylquinolizines according to claim 1 of the general formula

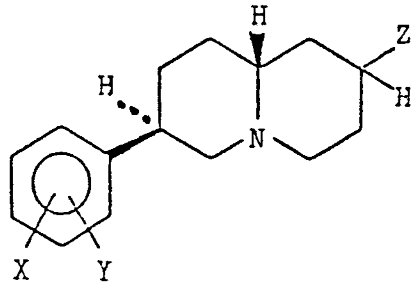

(VII)

wherein X, Y and Z signify the same as in claim 1, in the form of the racemate or of the enantiomers.

8. Phenylquinolizines according to claim 1 of the general formula

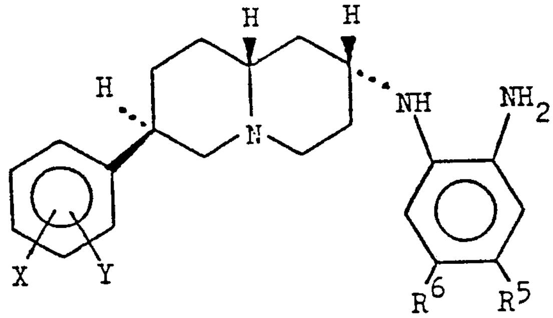

(X)

wherein X, Y, $R^5$ and $R^6$ signify the same as in claim 1, in the form of the racemate or of the enantiomers.

9. A compound according to claim 4, wherein X and Y represent hydrogen.

10. A compound according to claim 2, wherein X and Y represent hydrogen.

11. A compound according to claim 3, wherein X and Y represent hydrogen.

12. Rac-(9αβH)-7β-(o-fluorophenyl)octahydro-2α-(α′,α′,α′-trifluoro-2-amino-p-toluidino)-2H-quinolizine.

**Revendications**

1. Phénylquinolizines de formule générale

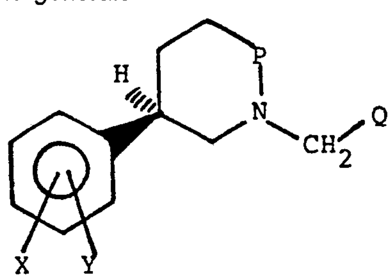

dans laquelle X représente l'hydrogène, le fluor, le chlore, un groupe alcoxy en $C_1$-$C_6$, alkyle en $C_1$-$C_6$ ou trifluorométhyle et Y représente l'hydrogène, le fluor, le chlore, un groupe alcoxy en $C_1$-$C_6$ ou alkyle en $C_1$-$C_6$ et P et Q forment ensemble l'un des groupes

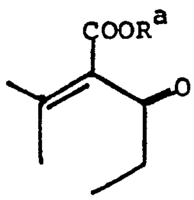
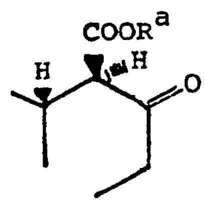
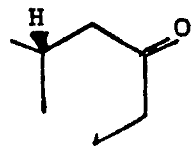
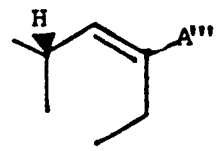

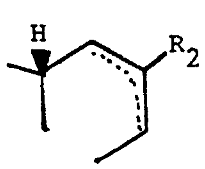
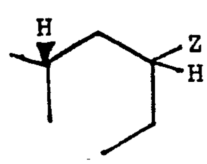
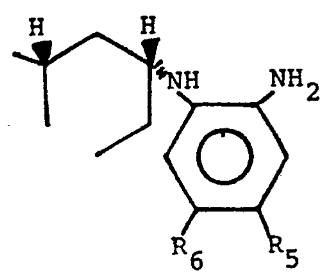

dans lesquels $R^a$ représente un groupe alkyle en $C_1$-$C_6$, $R^2$ représente un groupe alkyle en $C_1$-$C_6$, un groupe phényle éventuellement substitué par des halogènes, des groupes alcoxy en $C_1$-$C_6$ ou trifluorométhyle, $R^5$ et $R^6$ représentent chacun, indépendamment l'un de l'autre, l'hydrogène, un groupe méthyle, le fluor, le chlore, un groupe méthoxy ou trifluorométhyle, ou forment ensemble le groupe $-(CH_2)_4-$, Z représente un groupe éliminable et A''' représente un groupe

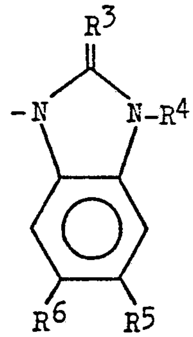

ou

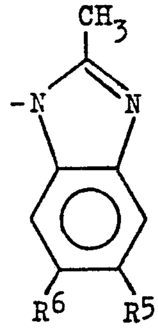

dans lequel $R^3$ à $R^6$ ont les significations suivantes :

$R^3$ représente l'oxygène ou le soufre,

$R^4$ représente l'hydrogène ou un groupe alkyle en $C_1$-$C_6$,

$R^5$ et $R^6$ représentent chacun, indépendamment l'un de l'autre : l'hydrogène, un groupe méthyle, le fluor, le chlore, un groupe méthoxy ou trifluorométhyle, ou forment ensemble le groupe $-(CH_2)_4$, étant spécifié que lorsque $R^4$ représente un groupe alkyle en $C_1$-$C_6$, $R^3$ représente l'oxygène, à l'état du racémate ou des énantiomères.

2. Phénylquinolizines selon la revendication 1 de formule générale

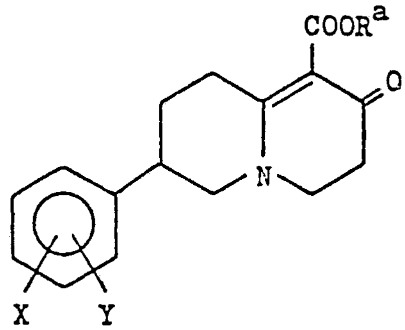

(XIII)

dans laquelle X, Y et $R^a$ ont les mêmes significations que dans la revendication 1.

3. Phénylquinolizines selon la revendication 1, de formule générale

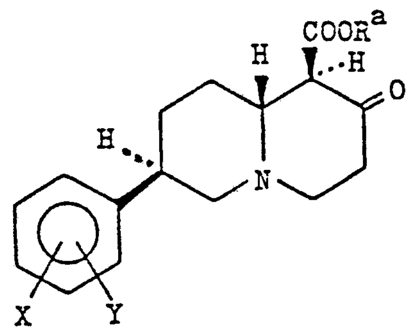

(XIV)

dans laquelle X, Y et $R^a$ ont les mêmes significations que dans la revendication 1, à l'état du racémate ou des énantiomères.

4. Phénylquinolizines selon la revendication 1 de formule générale

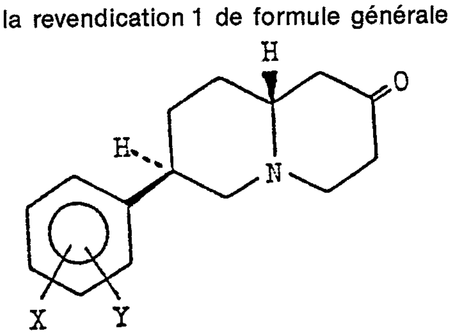

(V)

dans laquelle X et Y ont les mêmes significations que dans la revendication 1, à l'état du racémate ou des énantiomères.

5. Phénylquinolizines selon la revendication 1 de formule générale

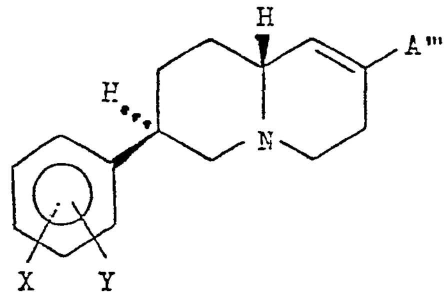

(IX) + (XV)

dans laquelle X, Y et A''' ont les mêmes significations que dans la revendication 1, à l'état du racémate ou des énantiomères.

6. Phénylquinolizines selon la revendication 1 de formule générale

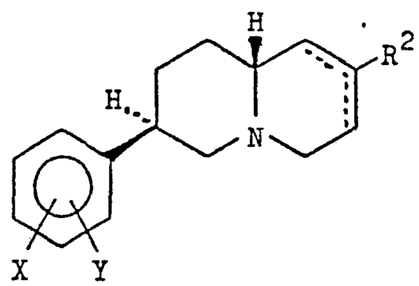

(VI)

dans laquelle X, Y et $R^2$ ont les mêmes significations que dans la revendication 1, et la liaison en traits interrompus représente une double liaison en position 1,2- ou 2,3-, à l'état du racémate ou des énantiomères.

7. Phénylquinolizines selon la revendication 1 de formule générale

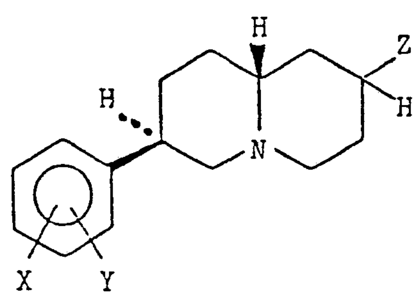

(VII)

dans laquelle X, Y et Z ont les mêmes significations que dans la revendication 1, à l'état du racémate ou des énantiomères.

8. Phénylquinolizines selon la revendication 1 de formule générale

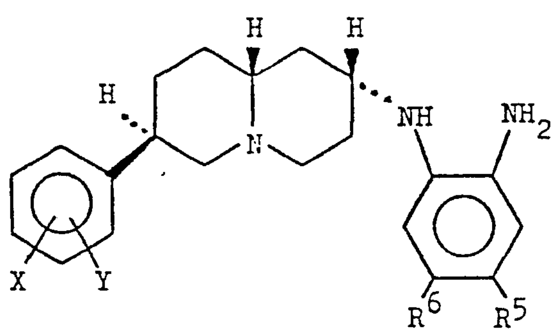

(X)

dans laquelle X, Y, $R^5$ et $R^6$ ont les mêmes significations que dans la revendication 1, à l'état du racémate ou des énantiomères.

9. Un composé selon la revendication 4, dans lequel X et Y représentent l'hydrogène.

10. Un composé selon la revendication 2, dans lequel X et Y représentent l'hydrogène.

11. Un composé selon la revendication 3, dans lequel X et Y représentent l'hydrogène.

12. La rac-(9αβH)-7β-(o-fluorophényl)octahydro-2α-(α',α',α'-trifluoro-2-amino-p-toluidino)-2H-quinolizine.